# EUROPEAN PATENT APPLICATION

(11) **EP 2 033 956 A1**
(43) Date of publication of application: **11.03.2009**
(21) Application number: 07115103.9
(22) Date of filing: 28.08.2007
(51) Int. Cl.: C07D 241/04, C07D 401/10, C07D 413/10, A61K 31/435, A61P 35/00

(54) **A new class of histone deacetylase inhibitors**

(71) Applicant: DAC S.r.l., 20121 Milano (IT)
(72) Inventor: Thaler, Florian, 21040 Gerenzano (IT); Varasi, Mario, 20142 Milano (IT); Mercurio, Ciro, 20025 Legnano (IT); Minucci, Saverio, 20090 Noversca Opera (IT); Colombo, Andrea, 20015 Parabiago (IT); Gagliardi, Stefania, 20059 Vimercate (IT)
(74) Representative: Gerli, Paolo

(57) **Abstract**

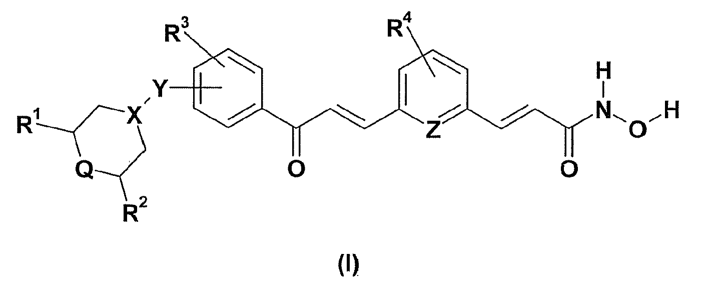

This invention is related to new histone deacetylase inhibitors according to the general formula (I) wherein: **Q** is a bond, CH₂, **NR⁵** or oxygen, **X** is CH or nitrogen, **Y** is a bond, CH₂, oxygen or **NR⁶**, **Z** is CH or nitrogen, **R¹, R²** are, independently, hydrogen or C₁-C₆ alkyl, **R³**, **R⁴** are, independently, hydrogen, halogen, C₁-C₆ alkyl, C₁-C₆ alkoxy, C₁-C₆ haloalkyl or C₁-C₆ haloalkoxy, and **R⁵** and **R⁶** are as further defined in the specification, and pharmaceutical acceptable salts thereof.

## Description

### FIELD OF THE INVENTION

The present invention relates to inhibitors of histone deacetylases (HDACs), to a process for their preparation, pharmaceutical compositions comprising them, and to their use as therapeutic agents, in particular for the treatment of cancer.

### BACKGROUND OF THE INVENTION

The reversible acetylation of the ε-amino groups of several lysine residues in the N-terminal histone tails mediates important conformational modifications in nucleosomes. These modifications influence the access of transcription factor to DNA and regulate gene expression (Davie, J.R. Curr. Opin. Genet. Dev. 1998, 8, 173-178). Two enzyme classes are involved in the process of acetylation and deacetylation of histones: histone acetyltransferases (HAT), which catalyse histone acetylation by acting as transcriptional co-activators, and histone deacetylase (HDAC).

After their recruitment to the promoter regions induced by transcription repressors and co-repressors such as Sin3, SMRT and N-CoR, histone deacetylases induce the formation of hypoacetylated histones and ultimately lead to transcriptional silencing (Wu, J. et al. Trends Biochem. Sci. 2000, 25, 619-623). The aberrant recruitment of histone deacetylases by oncogene proteins, or the disruption of the equilibrium between the activities of histone acetyltransferases and histone deacetylases are implicated in a series of pathologies, including:
1. Primarily, cancer (Lin, R.J. et al. Oncogene 2001, 20, 7204-7215; Kastner, P. et al. Oncogene 2001, 20, 7186-7203; Pandolfi, P. et al. Oncogene 2001, 20, 3116-3127; Grignani, F. et al. Nature 1998, 391, 815-818; Lutterbach, B. et al. Mol. Cell. Biol. 1998, 18, 7176-7184).
2. Non-tumor diseases:

### - CNS and PNS disorders:

- Huntington's disease (Ferrante, R.J. et al. J. Neurosci. 2003, 23, 9418-9427; Hockey, E. et al. Proc. Natl. Acad. Sci. USA 2003, 100, 2041-2046); bipolar disorders (Williams, R.S.B. et al. Nature 2002, 417, 292-295); cognitive disorders (Levenson, J.M. US20060018921); psychiatric disorders (Costa, E. et al. Crit. Rev. Neurabiol. 2003, 15, 121-142); fragile X syndrome (Chandler, S.P. et al. BMC Mol. Biol. 2003, 4, 3; Chiurazzi, P. et al. Hum. Mol. Genet. 1999, 8, 2317-2323) ;diseases caused by triplet expansion (Bodai, L. et al. Curr. Med. Chem. 2003, 10, 2577-2587; Hughes, R.E. Curr. Biol. 2002, 12, R141-143); neurodegenerative disorders (Jeong, M.R. et al. FEBS Lett. 2003, 542, 74-78); ischemia (Ming, R. et al. J. Neurochem. 2004, 89, 1358-1367); oxidative stress (Ryu, H. et al. Proc. Natl. Acad. Sci. USA 2003, 100, 4281-4286); inflammatory responses of the nervous system (Suuronen, T. J. Neurochem. 2003, 87, 407-416); epilepsy (Eyal, S. et al. Epilepsia 2004, 45, 737-744; Huang, Y. et al. J. Neurosci. 2002, 22, 8422-8428); diseases caused by protein aggregates (Corcoran, L.J. et al. Curr. Biol. 2004, 14, 488-492).
- Infections: HIV (Adam, E. et al. Mol. Cell. Biol. 2003, 23, 6200-6209; Van Lint, C. et al. Embo J. 1996, 15, 1112-1120; Demonte, D. et al. Biochem. Pharmacol. 2004, 68, 1231-1238; Ylisastigui, L. et al. Aids 2004, 18, 1101-1108); malaria, leishmaniasis, infections by protozoa, fungi, phytotoxic agents, viruses and parasites.
- Immune diseases: autoimmune diseases (Skov, S. et al. Blood 2003, 101, 1430-1438); chronic immune reactions against the host (Reddy, P. et al. Proc. Natl. Acad. Sci. USA 2004, 101, 3921-3926).
- Cardiovascular diseases: hypertrophy and cardiac decompensation (Kook, H. et al. J. Clin. Invest. 2003, 112, 863-871; McKinsey, T.A. et al. Novartis Found. Symp. 2004, 259, 132-141, discussion 141-145, 163-169; Hamamori, Y. et al. J. Clin. Invest. 2003, 112, 824-826).
- Muscular disorders: fibrotic skin disease (Rombouts, K. et al. Exp. Cell. Res. 2002, 278, 184-197); fibrosis (Niki, T. et al. Hepatology 1999, 29, 858-867); spinal and bulbar muscular atrophy (Minamiyama, M. et al. Hum. Mol. Genet. 2004, 13, 1183-1192).
- Other pathologies: arthritis (Chung, Y.L. et al. Mol. Ther. 2003, 8, 707-717); hyperlipidemia (Crestani, M. et al. WO05/105066); kidney diseases (Mishra, N. et al. J. Clin. Invest. 2003, 111, 539-552); psoriasis (McLaughlin, F. et al. Curr. Drug Targets Inflamm. Allergy 2004, 3, 213-219); intestinal and colitic diseases (Saemann, M.D. et al. Wien. Klin. Wochenschr. 2002, 114, 289-300); beta thalassemia (Rodgers, G.P. et al. Expert Opin. Investig. Drugs 2001, 10, 925-934); respiratory diseases (Barnes, P.J. Am. J. Respir. Crit. Care Med. 2003, 167, 813-818), Rubinstein-Taybi syndrome (Alarcon, J.M. et al. Neuron 2004, 42, 947-959).

In recent years there has been a considerable effort to develop inhibitors of histone deacetylases and several classes of compounds have been found to have potent and specific activities in preclinical studies. These classes include natural products (e.g. trichostatin A (TSA), trapoxin (TPX), depsipeptide (FK-228)), short chain fatty acids (sodium-butyrate, -phenylbutyrate and -valproate), hydroxamates (e.g. suberoylanilide (SAHA), pyroxamide, scriptaid, oxamflatin, LAQ824/LBH589) cyclic peptides containing hydroxamic acid (CHAPs) and benzamides (e.g. MS-275 and MGCD0103). All of them potently induce growth arrest, differentiation and apoptosis in a variety of transformed cells in culture as well as in animal models (Marks, P.A. et al. Curr. Opin. Oncol. 2001, 13, 477-483). Vorinostat (SAHA), romidepsin (depsipeptide, FK-228), belinostat (PXD101) and LBH589 showed therapeutic benefit as monotherapy in cutaneous T-cell lymphoma (CTCL) and also in other malignancies. The approval of the HDAC inhibitor vorinostat (Zolinza^{™}) was based on the inherent sensitivity of this type of lymphoma to alterations in acetylation patterns that resulted in the induction of repressed apoptotic pathways. (Johnstone, R.W. Nat. Rev. Drug Discov. 2002, 1, 287-299; Glaser, K.B. Biochemical Pharmacology 2007, 74, 659-671). Their clinical benefits, however, are limited by toxicity problems (TSA, CHAPs, MS-275), low stability (TSA), low solubility (TSA), poor potency and lack of selectivity (butyrates and analogues) (Vigushin, D. et al. Anti-Cancer Drugs 2002, 13, 1-13).

To overcome these liabilities many derivatives have been synthesised based on the structures of the aforementioned compounds, with some molecular substructures hypothesised by certain authors as being useful for the activity and penetration of cellular structures (Miller, T.A. Expert Opin. Ther. Patents 2004, 14, 791-804; Miller, T.A. J. Med. Chem. 2003, 46, 5098-5116; Moradei, O. et al. Curr. Med. Chem. - Anticancer Agents 2005, 5, 529-560; Minucci, S. et al. Nature Reviews Cancer, 2006 6, 38-51).

PCT application WO2006/037761 discloses HDAC inhibitors with the general formula 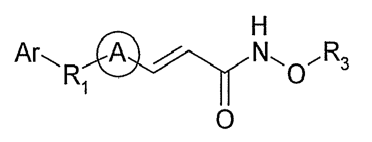 wherein **R₁** is a linear or branched chain, containing at least two conjugated double bonds, **R₃** is hydrogen or alkoxyalkyl; **Ar** is an optionally substituted aryl or heteroaryl group and **A** is a phenyl or pyridyl group, substituted by hydrogen, alkyl, cycloalkyl, aryl, arylalkyl, heterocyclyl, heterocyclylalkyl, halogen, haloalkyl, hydroxy, hydroxyalkyl, alkoxy, haloalkoxy, amino, aminoalkyl, alkylamino, (thio)carbonylamino, (thio)aminocarbonyl, sulphonylamino, aminosulphonyl, (thio)acyl, (thio)acyloxy, (thio)alkoxycarbonyl, nitro or nitryl; said patent application discloses 5 compounds wherein the **R₁** moiety is in meta position with respect to the acroyl hydroxamate group; no disclosure is present of compounds wherein **R₁** is in meta position with respect to the acroyl group and, at the same time, Ar is substituted by a saturated ring, either directly bound to the aryl group or linked through a spacer group. We have found now that certain meta substituted cinnamoyl and pyridyl-acryl hydroxamates are highly potent inhibitors of the HDAC enzyme.

### SUMMARY

According to the present invention there are provided compounds, endowed with a potent HDAC inhibitory activity, of general formula **(I)** 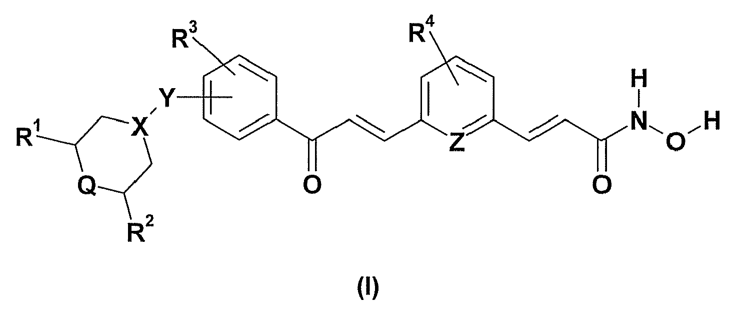 wherein:
- **Q**: is a bond, CH₂, **NR⁵** or oxygen;
- **X**: is CH or nitrogen;
- **Y**: is a bond, CH₂, oxygen or **NR⁶;**
- **Z**: is CH or nitrogen;
- **R¹, R²**: are, independently, hydrogen or C₁-C₆ alkyl;
- **R³, R⁴**: are, independently, hydrogen, halogen, C₁-C₆ alkyl, C₁-C₆ alkoxy, C₁- C₆haloalkyl or C₁-C₆ haloalkoxy;
- **R⁵**: is hydrogen, C₁-C₆ alkyl, (CO)**R⁷**, phenyl or benzyl;
- **R⁶**: is hydrogen, C₁-C₆ alkyl or benzyl;
- **R⁷**: is hydrogen, C₁-C₆ alkyl, phenyl, benzyl, O**R⁸** or **NR⁹R¹⁰;**
- **R⁸**: is C₂-C₆ alkyl;
- **R⁹**: is hydrogen, C₁-C₆ alkyl, phenyl or benzyl;
- **R¹⁰**: is hydrogen, C₁-C₆ alkyl or benzyl;
and the pharmaceutically acceptable salts thereof.

### DETAILED DESCRIPTION OF THE INVENTION

The phenyl or benzyl in **R⁵, R⁶, R⁷, R⁹,** and **R¹⁰** may be optionally substituted with one or more substituents selected from halogen, C₁-C₆ alkyl, C₁-C₆ haloalkyl, C₁-C₆ alkoxy, or C₁-C₆ haloalkoxy.

All the "alkyl" chains and alkyl-containing chains (e.g. haloalkyl) can be linear or branched.

"Halogens" are preferably fluorine, chlorine or bromine, being in particular fluorine or chlorine.

The "C₁-C₆ alkyl" group is preferably a linear or branched C₁-C₄ alkyl group, more preferably a C₁-C₂ alkyl group.

The "C₁-C₆ alkoxy" group is preferably a linear or branched C₁-C₄ alkoxy group, more preferably a C₁-C₂ alkoxy group.

The "C₁-C₆ haloalkyl" group is preferably a linear or branched C₁-C₄ haloalkyl group, more preferably a C₁-C₂ haloalkyl group, being in particular CF₃.

The "C₁-C₆ haloalkoxy" group is preferably a linear or branched C₁-C₄ haloalkoxy group, more preferably a C₁-C₂ haloalkoxy group, being in particular OCF₃, OCHF₂ or OCH₂F.

"Acceptable pharmaceutical salts" comprise salts obtained by salification with inorganic acids (e.g. hydrochloric, hydrobromide, sulphuric or phosphoric acids), or with organic acids (e.g. acetic, propionic, benzoic, cinnamic, mandelic, salicylic, glycolic, lactic, oxalic, malic, maleic, malonic, fumaric, tartaric, citric, p-toluenesulfonic or methanesulfonic acids).

In addition, the compounds of the present invention can exist in unsolvated as well as in solvated forms with pharmaceutically acceptable solvents such as water, ethanol and the like.

The compounds of the invention and their pharmaceutical acceptable salts can exist as single stereoisomers, racemates, and as mixtures of diastereoisomers. The compounds can exist also as geometric isomers. All such geometric isomers, single stereoisomers, racemates and mixtures thereof, are intended to be within the scope of the invention.

The present invention comprises metabolic precursors of formula **(I)** compounds. The term "metabolic precursors" means compounds having a different structure from that of the relevant formula **(I),** which after administration to the patient are directly or indirectly transformed into a compound of said formula **(I).** Methods for selecting metabolic precursors and their relative preparation are described for example in the book by Bundgaard (Bundgaard, H. ed., "Design of Prodrugs", Elsevier, 1985).

According to a first preferred embodiment this invention, in the above formula **(I):**
- **Q**: is a bond, CH₂, **NR⁵** or oxygen;
- **X**: is CH or nitrogen;
- **Y**: is a bond or CH₂;
- **Z**: is CH or nitrogen;
- **R¹, R²**: are, independently, hydrogen or C₁-C₄ alkyl;
- **R³, R⁴**: are, independently, hydrogen, halogen, C₁-C₄ alkyl, C₁-C₄ alkoxy, C₁- C₄ haloalkyl or C₁-C₄ haloalkoxy;
- **R⁵**: is hydrogen, C₁-C₄ alkyl, (CO)**R⁷**, phenyl or benzyl;
- **R⁷**: is hydrogen, C₁-C₄ alkyl, phenyl, benzyl, O**R⁸** or **NR⁹R¹⁰;**
- **R⁸**: is C₂-C₄ alkyl;
- **R⁹**: is hydrogen, C₁-C₄ alkyl, phenyl or benzyl;
- **R¹⁰**: is hydrogen, C₁-C₄ alkyl or benzyl;
and the pharmaceutically acceptable salts thereof.

According to a second preferred embodiment this invention, in the above formula (I):
- **Q**: is a bond, CH₂, **NR⁵** or oxygen;
- **X**: is CH or nitrogen;
- **Y**: is a bond or CH₂;
- **Z**: is CH or nitrogen;
- **R¹, R²**: are, independently, hydrogen or C₁-C₂ alkyl;
- **R³, R⁴**: are, independently, hydrogen, fluoro, chloro, C₁-C₄ alkyl, C₁-C₄ alkoxy, CF₃ or C₁-C₂ haloalkoxy;
- **R⁵**: is hydrogen, C₁-C₂ alkyl, (CO)**R⁷**, phenyl or benzyl;
- **R⁷**: is hydrogen, C₁-C₂ alkyl, phenyl, benzyl, O**R⁸** or **NR⁹R¹⁰;**
- **R⁸**: is C₂-C₄ alkyl;
- **R⁹**: is hydrogen, C₁-C₂ alkyl, phenyl or benzyl;
- **R¹⁰**: is hydrogen, C₁-C₂ alkyl or benzyl;
and the pharmaceutically acceptable salts thereof.

According to a third preferred embodiment this invention, in the above formula **(I):**
- **Q**: is **NR⁵** or oxygen;
- **X**: is nitrogen;
- **Y**: is a bond or CH₂
- **Z**: is CH or nitrogen;
- **R¹, R²**: are, independently, hydrogen or C₁-C₂ alkyl;
- **R³, R⁴**: are, independently, hydrogen, halogen, C₁-C₄ alkyl, C₁-C₄ alkoxy, C₁- C₄ haloalkyl or C₁-C₄ haloalkoxy;
- **R⁵**: is hydrogen, C₁-C₆ alkyl or (CO)CH₃;
and the pharmaceutically acceptable salts thereof.

Examples of specific compounds belonging to formula (I) are the following:
(E)-N-Hydroxy-3-(3-{(E)-3-[2-(4-methyl-piperazin-1-yl)-phenyl]-3-oxo-propenyl}-phenyl)-acrylamide;
(E)-N-Hydroxy-3-(3-{(E)-3-[2-(4-methyl-piperazin-1-ylmethyl)-phenyl]-3-oxo-propenyl}-phenyl)-acrylamide;
(E)-N-Hydroxy-3-(3-{(E)-3-[3-(4-methyl-piperazin-1-yl)-phenyl]-3-oxo-propenyl}-phenyl)-acrylamide;
(E)-3-(3-{(E)-3-[3-Chloro-5-(4-methyl-piperazin-1-yl)-phenyl]-3-oxo-propenyl}-phenyl)-N-hydroxy-acrylamide;
(E)-N-Hydroxy-3-(3-{(E)-3-[3-(4-methyl-piperazin-1-ylmethyl)-phenyl]-3-oxo-propenyl}-phenyl)-acrylamide;
(E)-N-Hydroxy-3-(3-{(E)-3-[4-(4-methyl-piperazin-1-yl)-phenyl]-3-oxo-propenyl}-phenyl)-acrylamide;
(E)-N-Hydroxy-3-(3-{(E)-3-[4-(4-methyl-piperazin-1-ylmethyl)-phenyl]-3-oxo-propenyl}-phenyl)-acrylamide;
(E)-N-Hydroxy-3-(6-{(E)-3-[2-(4-methyl-piperazin-1-yl)-phenyl]-3-oxo-propenyl}-pyridin-2-yl)-acrylamide;
(E)-N-Hydroxy-3-(6-{(E)-3-[2-(4-methyl-piperazin-1-ylmethyl)-phenyl]-3-oxo-propenyl}-pyridin-2-yl)-acrylamide;
(E)-N-Hydroxy-3-(6-{(E)-3-[3-(4-methyl-piperazin-1-yl)-phenyl]-3-oxo-propenyl}-pyridin-2-yl)-acrylamide;
(E)-3-(6-{(E)-3-[3-Chloro-5-(4-methyl-piperazin-1-yl)-phenyl]-3-oxo-propenyl}-pyridin-2-yl)-N-hydroxy-acrylamide;
(E)-N-Hydroxy-3-(6-{(E)-3-[3-(4-methyl-piperazin-1-ylmethyl)-phenyl]-3-oxo-propenyl}-pyridin-2-yl)-acrylamide;
(E)-N-Hydroxy-3-(6-{(E)-3-[4-(4-methyl-piperazin-1-yl)-phenyl]-3-oxo-propenyl}-pyridin-2-yl)-acrylamide;
(E)-N-Hydroxy-3-(6-{(E)-3-[4-(4-methyl-piperazin-1-ylmethyl)-phenyl]-3-oxo-propenyl}-pyridin-2-yl)-acrylamide;
(E)-3-(3-{(E)-3-[2-(4-Acetyl-piperazin-1-yl)-phenyl]-3-oxo-propenyl}-phenyl)-N-hydroxy-acrylamide;
(E)-3-(3-{(E)-3-[3-(4-Acetyl-piperazin-1-yl)-phenyl]-3-oxo-propenyl}-phenyl)-N-hydroxy-acrylamide;
(E)-3-(3-{(E)-3-[4-(4-Acetyl-piperazin-1-yl)-phenyl]-3-oxo-propenyl}-phenyl)-N-hydroxy-acrylamide;
(E)-3-(6-{(E)-3-[2-(4-Acetyl-piperazin-1-yl)-phenyl]-3-oxo-propenyl}-pyridin-2-yl)-N-hydroxy-acrylamide;
(E)-3-(6-{(E)-3-[3-(4-Acetyl-piperazin-1-yl)-phenyl]-3-oxo-propenyl}-pyridin-2-yl)-N-hydroxy-acrylamide;
(E)-3-(6-{(E)-3-[4-(4-Acetyl-piperazin-1-yl)-phenyl]-3-oxo-propenyl}-pyridin-2-yl)-N-hydroxy-acrylamide;
(E)-3-(3-{(E)-3-[2-(4-Acetyl-piperazin-1-ylmethyl)-phenyl]-3-oxo-propenyl}-phenyl)-N-hydroxy-acrylamide;
(E)-3-(3-{(E)-3-[3-(4-Acetyl-piperazin-1-ylmethyl)-phenyl]-3-oxo-propenyl}-phenyl)-N-hydroxy-acrylamide;
(E)-3-(3-{(E)-3-[4-(4-Acetyl-piperazin-1-ylmethyl)-phenyl]-3-oxo-propenyl}-phenyl)-N-hydroxy-acrylamide;
(E)-3-(6-{(E)-3-[2-(4-Acetyl-piperazin-1-ylmethyl)-phenyl]-3-oxo-propenyl}-pyridin-2-yl)-N-hydroxy-acrylamide;
(E)-3-(6-{(E)-3-[3-(4-Acetyl-piperazin-1-ylmethyl)-phenyl]-3-oxo-propenyl}-pyridin-2-yl)-N-hydroxy-acrylamide;
(E)-3-(6-{(E)-3-[4-(4-Acetyl-piperazin-1-ylmethyl)-phenyl]-3-oxo-propenyl}-pyridin-2-yl)-N-hydroxy-acrylamide;
(E)-3-(3-{(E)-3-[2-(4-Phenyl-piperazin-1-ylmethyl)-phenyl]-3-oxo-propenyl}-phenyl)-N-hydroxy-acrylamide;
(E)-3-(3-{(E)-3-[3-(4-Phenyl-piperazin-1-ylmethyl)-phenyl]-3-oxo-propenyl}-phenyl)-N-hydroxy-acrylamide;
(E)-3-(3-{(E)-3-[4-(4-Phenyl-piperazin-1-ylmethyl)-phenyl]-3-oxo-propenyl}-phenyl)-N-hydroxy-acrylamide;
(E)-3-(6-{(E)-3-[2-(4-Phenyl-piperazin-1-ylmethyl)-phenyl]-3-oxo-propenyl}-pyridin-2-yl)-N-hydroxy-acrylamide;
(E)-3-(6-{(E)-3-[3-(4-Phenyl-piperazin-1-ylmethyl)-phenyl]-3-oxo-propenyl}-pyridin-2-yl)-N-hydroxy-acrylamide;
(E)-3-(6-{(E)-3-[4-(4-Phenyl-piperazin-1-ylmethyl)-phenyl]-3-oxo-propenyl}-pyridin-2-yl)-N-hydroxy-acrylamide;
(E)-3-(3-{(E)-3-[2-(4-Benzyl-piperazin-1-yl)-phenyl]-3-oxo-propenyl}-phenyl)-N-hydroxy-acrylamide;
(E)-3-(3-{(E)-3-[3-(4-Benzyl-piperazin-1-yl)-phenyl]-3-oxo-propenyl}-phenyl)-N-hydroxy-acrylamide;
(E)-3-(3-{(E)-3-[4-(4-Benzyl-piperazin-1-yl)-phenyl]-3-oxo-propenyl}-phenyl)-N-hydroxy-acrylamide;
(E)-3-(6-{(E)-3-[2-(4-Benzyl-piperazin-1-yl)-phenyl]-3-oxo-propenyl}-pyridin-2-yl)-N-hydroxy-acrylamide;
(E)-3-(6-{(E)-3-[3-(4-Benzyl-piperazin-1-yl)-phenyl]-3-oxo-propenyl}-pyridin-2-yl)-N-hydroxy-acrylamide;
(E)-3-(6-{(E)-3-[4-(4-Benzyl-piperazin-1-yl)-phenyl]-3-oxo-propenyl}-pyridin-2-yl)-N-hydroxy-acrylamide;
(E)-3-(3-{(E)-3-[2-(4-Benzyl-piperazin-1-ylmethyl)-phenyl]-3-oxo-propenyl}-phenyl)-N-hydroxy-acrylamide;
(E)-3-(3-{(E)-3-[3-(4-Benzyl-piperazin-1-ylmethyl)-phenyl]-3-oxo-propenyl}-phenyl)-N-hydroxy-acrylamide;
(E)-3-(3-{(E)-3-[4-(4-Benzyl-piperazin-1-ylmethyl)-phenyl]-3-oxo-propenyl}-phenyl)-N-hydroxy-acrylamide;
(E)-3-(6-{(E)-3-[2-(4-Benzyl-piperazin-1-ylmethyl)-phenyl]-3-oxo-propenyl}-pyridin-2-yl)-N-hydroxy-acrylamide;
(E)-3-(6-{(E)-3-[3-(4-Benzyl-piperazin-1-ylmethyl)-phenyl]-3-oxo-propenyl}-pyridin-2-yl)-N-hydroxy-acrylamide;
(E)-3-(6-{(E)-3-[4-(4-Benzyl-piperazin-1-ylmethyl)-phenyl]-3-oxo-propenyl}-pyridin-2-yl)-N-hydroxy-acrylamide;
(E)-N-Hydroxy-3-(3-{(E)-3-oxo-3-[2-((3R,5S)-3,4,5-trimethyl-piperazin-1-yl)-phenyl]-propenyl}-phenyl)-acrylamide;
(E)-N-Hydroxy-3-(3-{(E)-3-oxo-3-[3-((3R,5S)-3,4,5-trimethyl-piperazin-1-yl)-phenyl]-propenyl}-phenyl)-acrylamide;
(E)-N-Hydroxy-3-(3-{(E)-3-oxo-3-[4-((3R,5S)-3,4,5-trimethyl-piperazin-1-yl)-phenyl]-propenyl}-phenyl)-acrylamide;
(E)-N-Hydroxy-3-(6-{(E)-3-oxo-3-[2-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-yl)-phenyl]-propenyl}-pyridin-2-yl)-acrylamide;
(E)-N-Hydroxy-3-(3-{(E)-3-oxo-3-[2-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[3-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[4-((3R,5S)-3,4,5-trimethyl-piperazin-1-ylmethyl)-phenyl]-propenyl}-phenyl)-acrylamide;
(E)-N-Hydroxy-3-(6-{(E)-3-oxo-3-[2-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-ylmethyl)-phenyl]-propenyl}-pyridin-2-yl)-acrylamide;
(E)-3-(3-{(E)-3-[2-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-yl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-yl)-phenyl]-3-oxo-propenyl}-pyridin-2-yl)-N-hydroxy-acrylamide;
(E)-3-(3-{(E)-3-[2-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-ylmethyl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-N-Hydroxy-3-{3-[(E)-3-(2-morpholin-4-yl-phenyl)-3-oxo-propenyl]-phenyl}-acrylamide;
(E)-N-Hydroxy-3-{3-[(E)-3-(3-morpholin-4-yl-phenyl)-3-oxo-propenyl]-phenyl} acrylamide;
(E)-N-Hydroxy-3-{3-[(E)-3-(4-morpholin-4-yl-phenyl)-3-oxo-propenyl]-phenyl}-acrylamide;
(E)-N-Hydroxy-3-{6-[(E)-3-(2-morpholin-4-yl-phenyl)-3-oxo-propenyl]-pyridin-2-yl}-acrylamide;
(E)-N-Hydroxy-3-{6-[(E)-3-(3-morpholin-4-yl-phenyl)-3-oxo-propenyl]-pyridin-2-yl}-acrylamide;
(E)-N-Hydroxy-3-{6-[(E)-3-(4-morpholin-4-yl-phenyl)-3-oxo-propenyl]-pyridin-2-yl}-acrylamide;
(E)-N-Hydroxy-3-{3-[(E)-3-(2-morpholin-4-ylmethyl-phenyl)-3-oxo-propenyl]-phenyl}-acrylamide;
(E)-N-Hydroxy-3-{3-[(E)-3-(3-morpholin-4-ylmethyl-phenyl)-3-oxo-propenyl]-phenyl}-acrylamide;
(E)-N-Hydroxy-3-{3-[(E)-3-(4-morpholin-4-ylmethyl-phenyl)-3-oxo-propenyl]-phenyl}-acrylamide;
(E)-N-Hydroxy-3-{6-[(E)-3-(2-morpholin-4-ylmethyl-phenyl)-3-oxo-propenyl]-pyridin-2-yl}-acrylamide;
(E)-N-Hydroxy-3-{6-[(E)-3-(3-morpholin-4-ylmethyl-phenyl)-3-oxo-propenyl]-pyridin-2-yl}-acrylamide;
(E)-N-Hydroxy-3-{6-[(E)-3-(4-morpholin-4-ylmethyl-phenyl)-3-oxo-propenyl]-pyridin-2-yl}-acrylamide;
(E)-N-Hydroxy-3-{3-[(E)-3-oxo-3-(2-piperazin-1-yl-phenyl)-propenyl]-phenyl}-acrylamide;
(E)-N-Hydroxy-3-{3-[(E)-3-oxo-3-(3-piperazin-1-yl-phenyl)-propenyl]-phenyl}-acrylamide;
(E)-N-Hydroxy-3-{3-[(E)-3-oxo-3-(4-piperazin-1-yl-phenyl)-propenyl]-phenyl}-acrylamide;
(E)-N-Hydroxy-3-{6-[(E)-3-oxo-3-(2-piperazin-1-yl-phenyl)-propenyl]-pyridin-2-yl}-acrylamide;
(E)-N-Hydroxy-3-{6-[(E)-3-oxo-3-(3-piperazin-1-yl-phenyl)-propenyl]-pyridin-2-yl}-acrylamide;
(E)-N-Hydroxy-3-{6-[(E)-3-oxo-3-(4-piperazin-1-yl-phenyl)-propenyl]-pyridin-2-yl}-acrylamide;
(E)-N-Hydroxy-3-{3-[(E)-3-oxo-3-(2-piperazin-1-ylmethyl-phenyl)-propenyl]-phenyl}-acrylamide;
(E)-N-Hydroxy-3-{3-[(E)-3-oxo-3-(3-piperazin-1-ylmethyl-phenyl)-propenyl]-phenyl}-acrylamide;
(E)-N-Hydroxy-3-{3-[(E)-3-oxo-3-(4-piperazin-1-ylmethyl-phenyl)-propenyl]-phenyl}-acrylamide;
(E)-N-Hydroxy-3-{6-[(E)-3-oxo-3-(2-piperazin-1-ylmethyl-phenyl)-propenyl]-pyridin-2-yl}-acrylamide;
(E)-N-Hydroxy-3-{6-[(E)-3-oxo-3-(3-piperazin-1-ylmethyl-phenyl)-propenyl]-pyridin-2-yl}-acrylamide;
(E)-N-Hydroxy-3-{6-[(E)-3-oxo-3-(4-piperazin-1-ylmethyl-phenyl)-propenyl]-pyridin-2-yl}-acrylamide;
(E)-3-(3-{(E)-3-[2-((3R,5S)-3,5-Dimethyl-piperazin-1-yl)-phenyl]-3-oxo-propenyl}-phenyl)-N-hydroxy-acrylamide;
(E)-3-(3-{(E)-3-[3-((3R,5S)-3,5-Dimethyl-piperazin-1-yl)-phenyl]-3-oxo-propenyl}-phenyl)-N-hydroxy-acrylamide;
(E)-3-(3-{(E)-3-[4-((3R,5S)-3,5-Dimethyl-piperazin-1-yl)-phenyl]-3-oxo-propenyl}-phenyl)-N-hydroxy-acrylamide;
(E)-3-(6-{(E)-3-[2-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-yl)-phenyl]-3-oxo-propenyl}-pyridin-2-yl)-N-hydroxy-acrylamide;
(E)-3-(3-{(E)-3-[2-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[3-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[4-((3R,5S)-3,5-Dimethyl-piperazin-1-ylmethyl)-phenyl]-3-oxo-propenyl}-phenyl)-N-hydroxy-acrylamide;
(E)-3-(6-{(E)-3-[2-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-ylmethyl)-phenyl]-3-oxo-propenyl}-pyridin-2-yl)-N-hydroxy-acrylamide.

The present invention also comprises the process for preparing the compounds of formula **(I).** The compounds of formula **(I)** can be obtained by the sequence outlined in Scheme 1: 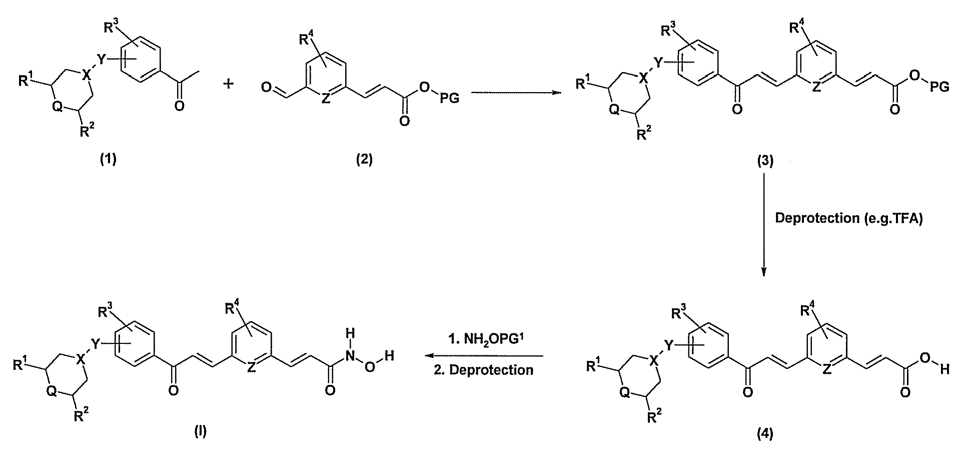 where **Q, X, Y, Z, R¹, R², R³, R⁴** are as defined above in formula **(I),** PG and PG¹ are protecting groups chosen among those known in the art, for example t-butyl, etc. for PG and O-(tetrahydro-2H-pyran-2-yl), etc. for PG¹.

The reaction between a compound of formula **(1)** and **(2)** can be carried out in presence of an inorganic base such as KOH or NaOH in a protic solvent, for example water, methanol, ethanol, or in THF at a temperature ranging from 0°C to room temperature. The compound of formula **(3)** can be deprotected into a compound of formula **(4)** according to known methods, e.g. by treatment of a t-butyl ester derivative with TFA (trifluoroacetic acid) in a suitable solvent such as dichloromethane at a temperature ranging from 0°C to room temperature.

The reaction of the compound of formula **(4)** with the protected hydroxylamine can be carried out with condensating agents such as EDC (1-(3-dimethylaminopropyl)-3-ethylcarbodiimide), in the presence of a suitable base (e.g. triethylamine or diisopropylethylamine) in a suitable solvent (e.g. tetrahydrofuran, dichloromethane or DMF). Generally an activator of the condensation reaction, such as HOBT (1-hydroxybenzotriazole) or HOAT (1-hydroxy-7-aza-benzotriazole), can be added to the reaction mixture. The reaction can be carried out at room temperature for a period lasting between about 2 and 12 h. Deprotection of the hydroxylamine, in the case of tetrahydropyranyl, can be achieved by known methods, for example using HCl in aprotic solvents (such as THF, diethylether or dioxane).

Compounds of formula **(1)** are known products or can be prepared with known methods by starting from known compounds. Compounds of formula **(2),** wherein **Z** is equal to CH, are known products or can be synthesized following the procedure described for compounds of formula **(IX)** in patent application WO2006/037761. Compounds of formula **(2),** wherein **Z** is equal to N, can be synthesized following the procedure described for compounds of formula **(XV)** in patent application WO2006/037761 or by reaction of the bromo-pyridine-carbaldehyde with *tert*-butylacrylate according the Heck reaction. The reaction conditions are described for example in the book by Larhed and Hallberg (Larhed, M.; Hallberg, A. "Handbook of Organopalladium Chemistry for Organic Synthesis", Negishi, E., Ed.; Wiley-Interscience, 2002). The reaction can be carried out in a suitable organic solvent (e.g. DMF) in the presence of palladium salts (e.g. palladium acetate), organic or inorganic bases (e.g. triethylamine, 1,4-diazabicyclo[2,2,2]-octane, sodium or potassium carbonate) and phosphine ligand derivatives, such as triphenylphosphine, at a temperature between room temperature and about 140°C.

In the case it is necessary to protect a chemical group of a compound of the present invention and/or an intermediate thereof, before carrying out one of the aforedescribed reactions, said chemical group can be protected and deprotected according to known methods. A thorough discussion for protection/deprotection steps is provided for example in Greene and Wuts (Greene, T.W.; Wuts, P.G.M. "Protective Groups in Organic Synthesis", John Wiley & Sons Inc., 1991) or in Kocienski (Kocienski, P.J. "Protecting Groups", George Thieme Verlag, 1994).

Salification of the compounds of formula **(I),** and the preparation of compounds of formula **(I),** free of their salts, can be carried out by known conventional methods.

The invention also comprises a method for preventing and/or treating diseases linked to the disregulation of histone deacetylase activity characterized by administering to a patient a pharmacologically useful quantity of one or more compounds of formula **(I),** as previously defined.

Diseases linked to the disregulation of histone deacetylase activity at which the present invention is aimed at are in particular tumor type diseases: e.g. leukemias and myeloid and lymphoid lymphomas, myelodysplastic syndromes, multiple myeloma, mammary tumors, pulmonary tumors and pleural mesotheliomas, skin tumors including basal cell carcinomas (basaliomas), melanomas, osteosarcomas, fibrosarcomas, rhabdomyosarcomas, neuroblastomas, glioblastomas, cerebral tumors, testicular and ovarian tumors, endometrial and prostate tumors, thyroid carcinomas, colorectal tumors, gastric tumors and gastrointestinal adenocarcinomas, hepatic carcinomas, pancreatic carcinomas, renal tumors, teratocarcinomas and embryonic carcinomas.

Non-tumor type diseases linked to the disregulation of histone deacetylase activity are for example Huntington's disease, diseases caused by triplet expansion, degenerative diseases, ischemia, oxidative stress, inflammatory responses of the nervous system, epilepsy, diseases caused by protein aggregates, HIV infections, malaria, leishmaniasis, infections by protozoa, fungi, phytotoxic agents, viruses and parasites, autoimmune diseases, chronic immune reactions against the host, hypertrophy and cardiac decompensation, fibrotic diseases of the skin, fibrosis, spinal and bulbar muscular atrophy, bipolar disorders, psychiatric disorders, fragile X syndrome, arthritis, renal diseases, psoriasis, intestinal and colitic diseases, beta thalassemia, respiratory diseases, Rubinstein-Taybi syndrome.

The compounds of formula **(I)** can also be used in combination with additional agents, in particular anti tumor and differentiating agents, either by separate administrations, or by including the two active principles in the same pharmaceutical formulation. Non comprehensive examples of suitable agents include retinoic acid, vitamin D; antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarabicin, mitomycin-C, dactinomycin, and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin); cytostatic agents such as antioestrogens (for example tamoxifen, toremifene, raloxifene, droloxifene and idoxifene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5-alpha-reductase such as finasteride; agents which inhibit cancer cell invasion (for example metalloproteinase inhibitors and inhibitors of urokinase plasminogen activator receptor function); inhibitors of growth factor function, for example such inhibitors include growth factor antibodies, growth factor receptor antibodies (for example the anti-erbb2 antibody trastuzumab [Herceptin^{™}] and the anti-erbbl antibody cetuximab), farnesyl transferase inhibitors, MEK inhibitors, tyrosine kinase inhibitors and serine/threonine kinase inhibitors, for example inhibitors of the epidermal growth factor family (for example gefitinib, erlotinib), antiangiogenic agents such as those which inhibit the effects of vascular endothelial growth factor, (for example the anti-vascular endothelial cell growth factor antibody bevacizumab [Avastin^{™}]), cell cycle inhibitors including for example CDK inhibitiors (for example flavopiridol, roscovitine) and other inhibitors of cell cycle checkpoints; inhibitors of aurora kinase and other kinases involved in mitosis and cytokinesis regulation; proteasome inhibitors (for example lactacystin and bortezomib); and other histone deacetylase inhibitors (for example SAHA, PXD101, LBH589, valproic acid, MS-275, MGCD0103 and FK-228).

The invention also comprises pharmaceutical compositions characterized by containing one or more active principles of formula **(I),** in association with pharmaceutically acceptable carrier, excipients and diluents.

The compounds of this invention can be administered via any of the accepted modes of administration or agents for serving similar utilities. Thus, administration can be, for example, oral, nasal, parental (intravenous, subcutaneous, intramuscular), including buccal, sublingual, rectal, topical, transdermal, intravescial, or using any other route of administration.

The compounds of formula **(I)** can be pharmaceutically formulated according to known methods. The pharmaceutical compositions can be chosen on the basis of the treatment requirements. Such compositions are prepared by blending and are suitably adapted to oral or parenteral administration, and as such can be administered in the form of tablets, capsules, oral preparations, powders, granules, pills, injectable or infusible liquid solutions, suspensions or suppositories.

Tablets and capsules for oral administration are normally presented in unit dose form and contain conventional excipients such as binders, fillers, diluents, tableting agents, lubricants, detergents, disintegrants, coloring agents, flavoring agents and wetting agents. The tablets can be coated using methods well known in the art.

Suitable fillers include cellulose, mannitol. lactose and other similar agents. Suitable disintegrants include polyvinylpyrrolidone and starch derivatives such as sodium glycolate starch. Suitable lubricants include, for example, magnesium stearate. Suitable wetting agents include sodium lauryl sulfate.

These oral solid compositions can be prepared by conventional methods of blending, filling or tableting. The blending operation can be repeated to distribute the active principle throughout compositions containing large quantities of fillers. Such operations are conventional.

Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatin, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavoring or coloring agents.

Oral formulations also include conventional slow-release formulations such as enterically coated tablets or granules.

For parenteral administration, fluid unit dosages can be prepared, containing the compound and a sterile vehicle. The compound can be either suspended or dissolved, depending on the vehicle and concentration. The parenteral solutions are normally prepared by dissolving the compound in a vehicle, sterilising by filtration, filling suitable vials and sealing.. Advantageously, adjuvants such as local anaesthetics, preservatives and buffering agents can also be dissolved in the vehicle. To increase stability, the composition can be frozen after having filled the vials and removed the water *in vacuo*. Parenteral suspensions are prepared in substantially the same manner, except that the compound can be suspended in the vehicle instead of being dissolved, and sterilized by exposure to ethylene oxide before suspending in the sterile vehicle. Advantageously, a surfactant or wetting agent can be included in the composition to facilitate uniform distribution of the compound of the invention.

Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. A reference for the formulations is the book by Remington ("Remington: The Science and Practice of Pharmacy", Lippincott Williams & Wilkins, 2000). Creams, as known to formulation experts, are viscous liquids or semisolid emulsions, and contain an oil phase, an emulsifier and an aqueous phase. The oil phase generally contains petrolatum and an alcohol such as cetyl or stearic alcohol. The emulsifier in a cream formulation is chosen from non-ionic, anionic, cationic or amphoteric surface-active agents. The monophasic gels contain the organic molecules uniformly distributed in the liquid, which is generally aqueous, but they also preferably contain an alcohol and optionally an oil. Preferred gelling agents are cross-linked acrylic acid polymers (e.g. carbomer-type polymers, such as carboxypolyalkylenes, which are commercially available under the Carbopol^{™} trademark). Hydrophilic polymers are also preferred, such as polyoxyethylene, polyoxyethylene-polyoxypropylene copolymers and polyvinyl alcohol; cellulose polymers such as hydroxypropyl cellulose, hydroxyethyl cellulose, hydroxypropyl methylcellulose, hydroxypropyl methylcellulose phthalate and methylcellulose; gums, such as xanthan gum and tragacanth gum; sodium alginate; and gelatin. Dispersing agents such as alcohol or glycerin can be added for gel preparation. The gelling agent can be dispersed by finely chopping and/or mixing.

A further method of administering the compounds of the invention regards transdermal delivery. Typical transdermal formulations comprise conventional aqueous and non-aqueous vectors, such as creams, oils, lotions or pastes or can be in the form of membranes or medicated patches. One formulation provides that a compound of the invention is dispersed within a pressure sensitive patch which adheres to the skin. This formulation enables the compound to diffuse from the patch to the patient through the skin. For a constant release of the drug through the skin, natural rubber and silicon can be used as pressure sensitive adhesives. The above mentioned uses and methods also include the possibility of coadministration of additional therapeutic agents, simultaneously or delayed with respect to the administration of the compound of formula (I).

In the previously mentioned uses and methods, the dosage of the compounds of formula (I), can vary depending upon a variety of factors including the patient type and condition, the degree of disease severity, mode and time of administration, diet and drug combinations. As an indication, they can be administered within a dose range of between 0.001 and 1000 mg/kg/day. The determination of optimum dosages for a particular patient is well known to one skilled in the art.

As is common practice, the compositions are normally accompanied by written or printed instructions for use in the treatment in question.

The following examples serve to provide further appreciation of the invention, but are not meant in any way to restrict the scope of the invention.

### EXPERIMENTAL PART

### 1. CHEMICAL SYNTHESIS

### Methods

Unless otherwise indicated, all the starting reagents were found to be commercially available and were used without any prior purification. Specifically, the following abbreviations may have been used in the descriptions of the experimental methods.

| g (grams) | NMR (Nuclear Magnetic Resonance) |
|---|---|
| mg (milligrams) | ¹H (proton) |
| ml (millilitres) | MHz (Megahertz) |
| M (molarity) | Hz (Hertz) |
| µl (microlitres) | LC-MS (Liquid Chromatography Mass Spectrum) |
| mmol (millimoles) | rt (retention time in minutes) |
| RT (room temperature) | s (seconds) |
| min (minutes) | h (hours) |
| THF (tetrahydrofuran) | MeOH (methanol) |
| EtOH (ethanol) | *i*-PrOH (isopropyl alcohol) |
| EtOAc (ethyl acetate) | Et₂O (diethyl ether) |
| *i*-Pr₂O (diisopropylether) | AcOH (acetic acid) |
| DCM (dichloromethane) | DMSO (dimethyl sulfoxide) |
| DMSO-d₆ (deuterated dimethyl sulfoxide) | DMF (dimethylformamide) |
| K₂CO₃ (potassium carbonate) | NaOH (sodium hydroxide) |
| NH₄OH (ammonium hydroxide) | KOH (potassium hydroxide) |
| Na₂SO₄ (sodium sulphate) Pd(OAc)₂ (palladium acetate) | HCl (hydrochloric acid) NaBH(OAc)₃ (sodium triacetoxyborohydride) |
| TFA (trifluoroacetic acid) | TEA (triethylamine) |
| EDC (1-3(dimethylaminopropyl)-3-ethylcarbodiimide hydrochloride) | HOBT (1-hydroxybenzotriazole) |
| NH₂OTHP (O-(tetrahydro-2H-pyran-2-yl)hydroxylamine) | BOC (*tert*-butoxycarbonyl) |

Except where indicated otherwise, all temperatures are expressed in °C (degrees centigrade).

The ¹H-NMR spectra were acquired with a Brucker 300 MHz. The chemical shifts are expressed in parts per million (ppm, δ units). The coupling constants are expressed in Hertz (Hz) and the splitting patterns are described as s (singlet), d (doublet), t (triplet), q (quartet), quint (quintet), m (multiplet), bs (broad singlet).

The LC-MS analyses were undertaken in accordance with the following methods: METHOD A: Waters Acquity UPLC, Micromass ZQ Single quadrupole (Waters). Column Acquity UPLC-BEH C18 (50x2.1 mm, 1.7 µm);

Flow rate: 0.6 ml/min splitting ratio MS:waste/1:4;

Mobile phase: A phase=water/CH₃CN 95/5 +0.1% TFA; B phase=water/CH₃CN 5/95 +0.1% TFA. 0-0.25 min (A: 95%, B: 5%), 0.25-3.30 min (A: 0%, B: 100%), 3.30-4.00 min (A: 0%, B: 100%), 4.00-4.10 min (A: 95%, B: 5%); 4.10-5.00 min (A: 95%, B: 5%). UV detection wavelength 254 nm or BPI; Injection volume: 2µl

METHOD B: Waters Acquity UPLC, Micromass ZQ Single quadrupole (Waters). Column Acquity UPLC-BEH C18 (50x2.1 mm, 1.7 µm);

Flow rate: 0.6 ml/min splitting ratio MS:waste/1:4;

Mobile phase: A phase=water/CH₃CN 95/5 +0.1% TFA; B phase=water/CH₃CN 5/95 + 0.1% TFA. 0-0.5 min (A: 95%, B: 5%), 0.5-6 min (A: 0%, B: 100%), 6.00-7.00 min (A: 0%, B: 100%), 7.00-7.10 min (A: 95%, B: 5%); 7.10-8.50 min (A: 95%, B: 5%). UV detection wavelength 254 nm or BPI; Injection volume: 2µl

METHOD C: Waters Acquity UPLC, Micromass ZQ Single quadrupole (Waters). Column Acquity UPLC-BEH C18 (50x2.1 mm, 1.7 µm);

Flow rate: 0.6 ml/min splitting ratio MS:waste/1:4;

Mobile phase: A phase=water/CH₃CN 95/5 + 0.1% TFA; B phase=water/CH₃CN 5/95 +0.1% TFA. 0-0.25 min (A: 98%, B: 2%), 0.25-3.30 min (A: 0%, B: 100%), 3.30-4.00 min (A: 0%, B: 100%), 4.00-4.10 min (A: 98%, B: 2%); 4.10-5.00 min (A: 98%, B: 2%). UV detection wavelength 254 nm or BPI; Injection volume: 2µl

All the mass spectra were acquired with the ESI method.

Most of the reactions were monitored by thin layer chromatography (TLC) with 0.2 mm Merck silica gel plates (60F-254), visualized with UV light (254 nm). The chromatographic columns were packed with Merck silica gel 60 (0.04-0.063 mm).

### Preparation 1: tert-Butyl (E)-3-(3-formyl-phenyl)-acrylate

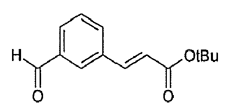

3-Bromo-benzaldehyde (5 g, 27 mmol) was dissolved in DMF (70 ml) and TEA (8.5 ml, 61 mmol). To the resulting solution PPh₃ (354 mg, 1.35 mmol), Pd(OAc)₂ (121 mg, 0.54 mmol), NaHCO₃ (4.5 g, 54 mmol) and *tert*-butyl acrylate (3.97 ml, 27 mmol) were added under nitrogen and heated to 100°C for 3 h. Additional Pd(OAc)₂ (60 mg, 0.26 mmol) was added and after 1 h at 100°C the slurry was partitioned between water and Et₂O. The organic phase was dried over Na₂SO₄ and evaporated *under vacuum.* The crude reaction mixture was purified by column chromatography (eluent: petroleum ether/AcOEt 95:5) to give *tert*-butyl (E)-3-(3-formyl-phenyl)-acrylate (3.5 g) as a pale yellow oil.
Y=56 %

### Preparation 2: 1-[2-(4-Methyl-piperazin-1-yl)-phenyl]-ethanone

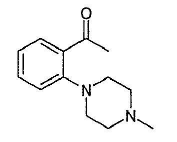

### STEP A

A mixture of 2-fluoro benzonitrile (2.28 g, 18.84 mmol), 1-methyl piperazine (3.14 ml, 28.26 mmol) and finely grounded K₂CO₃ (3.19 g, 23 mmol) in DMSO (50 ml) was heated to 120°C for 24 h.

The mixture was then diluted with H₂O and extracted twice with AcOEt. The collected organic phases were dried over Na₂SO₄ and evaporated to dryness. The crude product was purified by column chromatography (eluent: DCM/MeOH/NH₄OH 97:3:0.3) and the obtained compound was dissolved in DCM and treated with HCl in Et₂O. The resulting precipitate was filtered and rinsed with DCM to give 2-(4-methyl-piperazin-1-yl)-benzonitrile hydrochloride (3.15 g).
Y=70%

### STEP B

A solution of 2-(4-methyl-piperazin-1-yl)-benzonitrile hydrochloride (2.16 g, 9.1 mmol) in H₂O was brought to basic pH with NH₄OH and extracted with DCM. The organic phase was dried over Na₂SO₄, and evaporated *under vacuum.* The resulting 2-(4-methyl-piperazin-1-yl)-benzonitrile (1.77 g, 8.80 mmol) was dissolved in 30 ml of toluene and added under nitrogen atmosphere to a 3 M solution of methyl magnesium bromide in Et₂O (8.79 ml, 26.38 mmol). The resulting suspension was heated to reflux for 4 h. The reaction was cooled down to 0°C, acidified with 10% HCl and then heated to reflux for 1 h. The two layers were separated and the aqueous phase was extracted with AcOEt, then basified with NH₄OH and extracted with DCM. The organic phase was dried over Na₂SO₄ and concentrated to dryness. The crude mixture was purified by column chromatography (DCM/MeOH/NH₄OH 98:2:0.2) to give 1-[2-(4-methyl-piperazin-1-yl)-phenyl]-ethanone (1.62 g).
Y=84%

### Preparation 3: 1-[2-(4-Methyl-piperazin-1-yl-methyl)-phenyl]-ethanone

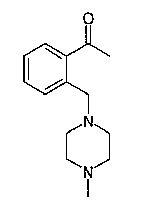

A mixture of 2-acetyl-benzaldehyde (1 g, 6.75 mmol), N-methyl piperazine (878 mg, 8.76 mmol) and NaBH(OAc)₃ (2.14 g, 10.12 mmol) in DCM (50 ml) was stirred at RT for 1 h and then acetic acid (526 mg, 8.76 mmol) was added. The resulting solution was stirred at RT overnight, then diluted with DCM and finally washed with 1 M Na₂CO₃. The organic phase was dried over Na₂SO₄ and evaporated *under vacuum.* The crude mixture was purified by column chromatography (eluent: DCM/MeOH/NH₄OH 95:5:0.2) to give 1-[2-(4-methyl-piperazin-1-yl-methyl)-phenyl]-ethanone (1.06 g).
Y=67%

### Preparation 4: 1-[3-(4-Methyl-piperazin-1-yl-methyl)-phenyl]-ethanone

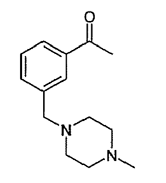

### STEP A

A mixture of 3-formyl-benzonitrile (1.5 g, 11.45 mmol), N-methyl piperazine (1.49 g, 14.9 mmol) and NaBH(OAc)₃ (3.63 g, 17.18 mmol) in DCM (75 ml) and AcOH (0.851 ml, 14.9 mmol) was stirred at RT overnight and then diluted with DCM and washed with 1 M Na₂CO₃. The organic phase was dried over Na₂SO₄ and evaporated *under vacuum.* The crude product was purified by column chromatography (eluent: DCM/MeOH/NH₄OH 97:3:0.5) to give 3-(4-methyl-piperazin-1-yl-methyl)-benzonitrile (1.7 g).
Y=70%

### STEP B

A solution of 3-(4-methyl-piperazin-1-yl-methyl)-benzonitrile (1.7 g, 7.91 mmol) in dry toluene (20 ml) was added dropwise to a stirred 3 M solution of methyl magnesium bromide in diethyl ether (7.91 ml, 23.72 mmol) under nitrogen at 0°C. The mixture was heated to 80°C for 6 h and then stirred overnight at RT. The resulting slurry was treated with 10% HCl and ice for 1 h and then basified with 1 M NaOH and extracted with AcOEt. The organic phase was dried over Na₂SO₄ and evaporated *under vacuum.* The crude product was purified by column chromatography (eluent: DCM/MeOH/NH₄OH from 97:3:0.1 to 95:5:0.2) to give 1-[3-(4-methyl-piperazin-1-yl-methyl)-phenyl]-ethanone (1.69 g).
Y=92%

### Preparation 5: 1-[4-(4-methyl-piperazin-1-yl-methyl)-phenyl]-ethanone

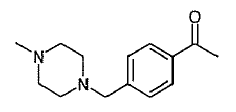

### STEP A

N-Methyl piperazine (0.805 ml, 7.6 mmol) was added to a solution of 4-(bromomethyl)benzonitrile (1 g, 5.1 mmol) and TEA (1.4 ml, 10.2 mmol) in DCM (15 ml) and the resulting mixture was stirred at RT for 24 h. The solution was then diluted with DCM, washed with a 5% NaHCO₃ solution and then with H₂O. The organic phase was dried over Na₂SO₄ and evaporated to dryness to give 4-(4-methyl-piperazin-1-yl-methyl)-benzonitrile (0.73 g) as a white solid.
Y=67%

### STEP B

4-(4-Methyl-piperazin-1-yl-methyl)-benzonitrile (0.73 g, 3.40 mmol) was dissolved in toluene (13 ml) and added under inert atmosphere to a stirred 3 M solution of methyl magnesium bromide in Et₂O (3.4 ml, 10.2 mmol). The resulting suspension was heated to reflux for 4 h. The reaction was cooled down to 0°C, acidified with 10% HCl and then refluxed at 100°C for 1 h. The layers were separated and the aqueous phase extracted with AcOEt, basified with NH₄OH and extracted with DCM. The latter organic phase was dried over Na₂SO₄ and evaporated to dryness to yield 1-[4-(4-methyl-piperazin-1-yl-methyl)-phenyl]-ethanone (0.71 g) as a yellow oil.
Y=90%

### Preparation 6: 1-[3-Chloro-5-(4-methyl-piperazin-1-yl)-phenyl]-ethanone

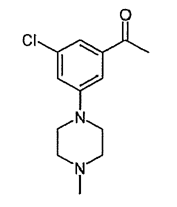

### STEP A

A mixture of 3-chloro-5-fluoro-benzonitrile (1.1 g, 7.07 mmol), 1-methyl-piperazine (1.18 ml, 10.6 mmol), and K₂CO₃ (2.92 g, 21.21 mmol) in DMSO (25 ml) was heated to 100°C overnight and then partitioned between water and Et₂O. The aqueous phase was extracted with Et₂O and the collected organic phases were dried over Na₂SO₄ and evaporated *under vacuum.* The residue was dissolved in Et₂O and extracted with 0.5 M HCl. The aqueous phase was basified with NH₄OH and extracted with DCM. The organic phase was dried over Na₂SO₄ and evaporated to give 1.01 g of 3-chloro-5-(4-methyl-piperazin-1-yl)-benzonitrile.
Y=61%

### STEP B

A solution of 3-chloro-5-(4-methyl-piperazin-1-yl)-benzonitrile (1.01 g, 4.3 mmol) in toluene (10 ml) was added dropwise at 0°C to a stirred 3 M solution of methyl magnesium bromide in Et₂O (4.3 ml, 12.9 mmol) under nitrogen.

The mixture was heated to 80°C for 4 h and then treated with 10% HCl. The resulting mixture was stirred at RT for 30 min and then the layers were separated. The aqueous phase was extracted with AcOEt, then basified with K₂CO₃ and extracted twice with AcOEt. The collected organic phases were dried over Na₂SO₄ and evaporated *under vacuum.* The crude product was purified by column chromatography (eluent: DCM/MeOH/NH₄OH 97:3:0.1) to give 1-[3-chloro-5-(4-methyl-piperazin-1-yl)-phenyl]-ethanone (0.723 g) as a yellow solid.
Y=66 %

### Preparation 7: tert-Butyl (E)-3-(6-formyl-pyridin-2-yl)-acrylate

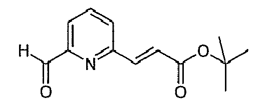

A mixture of 6-bromo-pyridine-2-carbaldehyde (205 mg, 1.1 mmol), *tert*-butyl acrylate (0.483 ml, 3.3 mmol), Pd(OAc)₂ (5 mg, 0.022 mmol), 1,4-diazabicyclo[2.2.2]octane (5 mg, 0.044 mmol), n-Bu₄NBr (354 mg, 1.1 mmol) and K₂CO₃ (152 mg, 1.1 mmol) in DMF (3 ml) was heated to 120°C for 5 h under MW irradiation. The solvent was then removed *under vacuum* and the crude mixture was purified by column chromatography (eluent: petroleum ether/AcOEt 10:1) to give *tert*-butyl (E)-3-(6-formyl-pyridin-2-yl)-acrylate (0.134 g) as white solid.
Y=52%

### Example 1: (E)-N-Hydroxy-3-(3-{(E)-3-[2-(4-methyl-piperazin-1-yl)-phenyl]-3-oxo-propenyl}-phenyl)-acrylamide

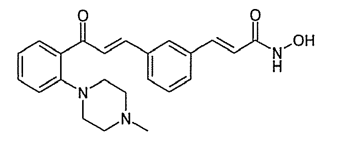

### STEP A

A mixture of *tert*-butyl (E)-3-(3-formyl-phenyl)-acrylate (prepared as described in Preparation 1, 220 mg, 0.95 mmol), 1-[2-(4-methyl-piperazin-1-yl)-phenyl]-ethanone (prepared as described in Preparation 2, 200 mg, 0.92 mmol) and KOH (102 mg, 1.82 mmol) in EtOH (5 ml) and H₂O (1 ml), was stirred at RT for 12 h.

The mixture was then partitioned between water and AcOEt and the organic phase was dried over Na₂SO₄ and evaporated *under vacuum.* The crude product was dissolved in DCM (2 ml) and TFA (1 ml) and the resulting solution was stirred for 6 h at RT. The solvent was then removed *under vacuum* to give of (E)-3-(3-{(E)-3-[2-(4-methyl-piperazin-1-yl)-phenyl]-3-oxo-propenyl}-phenyl)-acrylic acid (0.220 g) as trifluoroacetate salt.
Y=49%

### STEP B

A mixture of (E)-3-(3-{(E)-3-[2-(4-methyl-piperazin-1-yl)-phenyl]-3-oxo-propenyl}-phenyl)-acrylic acid trifluoroacetate (220 mg, 0.45 mmol), HOBT (95 mg, 0.702 mmol), EDC (134 mg, 0.70 mmol), TEA (0.245 ml, 1.75 mmol) and NH₂OTHP (68 mg, 0.58 mmol) in THF (5 ml) and DMF (1 ml), was stirred for 12 h at RT and then partitioned between water and AcOEt. The organic extract was dried over Na₂SO₄, evaporated *under vacuum* and the crude product was purified by column chromatography (eluent: AcOEt to AcOEt/MeOH 9:1). The collected fractions were evaporated and the resulting compound was dissolved in DCM and treated with HCl/Et₂O for 2 h. The precipitate was filtered and purified by preparative LC-MS to give (E)-N-hydroxy-3-(3-{(E)-3-[2-(4-methyl-piperazin-1-yl)-phenyl]-3-oxo-propenyl}-phenyl)-acrylamide (0.053 g) as its trifluoroacetate salt.
Y=23%
LC-MS: Method C, rt=1.4; (ES+) MH⁺: 392.3
¹H NMR (DMSO-d₆) δ (ppm): 10.75 (bs, 1 H), 9.69 (bs, 1 H), 7.98 (s, 1 H), 7.79 (d, 1 H), 7.65 (d, 1 H), 7.43 - 7.60 (m, 6 H), 7.29 (d, 1 H), 7.21 (td, 1 H), 6.55 (d, 1 H), 3.23 - 3.53 (m, 4 H), 3.02 - 3.22 (m, 2 H), 2.84 - 3.00 (m, 2 H), 2.71 (s, 3 H).

### Example 2: (E)-N-Hydroxy-3-(3-{(E)-3-[2-(4-methyl-piperazin-1-yl-methyl)-phenyl]-3-oxo-propenyl}-phenyl)-acrylamide

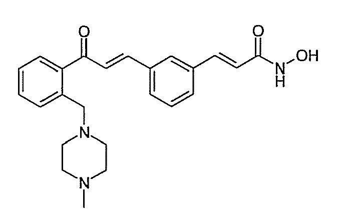

1.7 M KOH (0.634 ml) was added dropwise to a stirred mixture of 1-[2-(4-methyl-piperazin-1-yl-methyl)-phenyl]-ethanone (prepared as described in Preparation 3, 250 mg, 1.08 mmol) and *tert*-butyl (E)-3-(3-formyl-phenyl)-acrylate (prepared as described in Preparation 1, 250 mg, 1.08 mmol) in EtOH (15 ml) at 0°C. The resulting mixture was stirred at 0°C for 4 h and then partitioned between water and AcOEt. The organic phase was dried over Na₂SO₄ and evaporated to dryness. The crude product was purified by column chromatography (eluent: petroleum ether/AcOEt 7:3 and then DCM/MeOH/NH₄OH 95:5:0.1) to give *tert*-butyl (E)-3-(3-{(E)-3-[2-(4-methyl-piperazin-1-yl-methyl)-phenyl]-3-oxo-propenyl}-phenyl) acrylate (0.384 g).
Y=80%

### STEP B

A mixture of *tert*-butyl (E)-3-(3-{(E)-3-[2-(4-methyl-piperazin-1-yl-methyl)-phenyl]-3-oxo-propenyl}-phenyl)-acrylate (384 mg, 0.861 mmol) and TFA (1.5 ml) in DCM (6 ml) was stirred for 6 h at RT. The solvent was removed *under vacuum* and the residue was dissolved in DMF (15 ml). HOBT (232 mg, 1.72 mmol), EDC (328 mg, 1.71 mmol), TEA (0.239 ml, 1.72 mmol) and NH₂OTHP (121 mg, 1.03 mmol) were added and the resulting mixture was stirred at RT for 5 h. Then water was added, and the product was extracted with AcOEt followed by DCM. The collected organic phases were dried over Na₂SO₄ and evaporated *under vacuum.* The crude product was purified by column chromatography (eluent: DCM/MeOH/NH₄OH 97:3:0.1) and the resulting product was dissolved in DCM and treated with HCl/Et₂O for 2 h. The precipitate was filtered, crystallized from i-PrOH/MeOH/diisopropyl ether and then purified by preparative LC-MS to give (E)-N-hydroxy-3-(3-{(E)-3-[2-(4-methyl-piperazin-1-yl-methyl)-phenyl]-3-oxo-propenyl}-phenyl)-acrylamide as its bis-trifluoroacetate salt (0.124 g).
Y=23 %
LC-MS: Method B, rt=1.66; (ES+) MH⁺: 406.20
¹H NMR (DMSO-d₆ 353K) δ (ppm): 7.91 (s, 1 H), 7.72 (d, 1 H), 7.38 - 7.68 (m, 9 H), 6.65 (d, 1 H), 3.97 (s, 2 H), 3.08 - 3.36 (m, 4 H), 2.93 (bs, 4 H), 2.69 (s, 3 H).

### Example 3: (E)-N-Hydroxy-3-(3-{(E)-3-[3-(4-methyl-piperazin-1-yl)-phenyl]-3-oxo-propenyl}-phenyl)-acrylamide

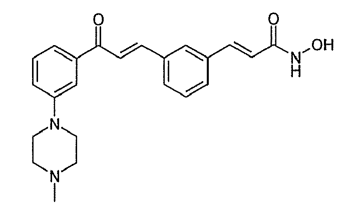

### STEP A

A solution of 1-[3-(4-methyl-piperazin-1-yl)-phenyl]-ethanone (350 mg, 1.6 mmol) in EtOH (5 ml) was added within 2 h to a stirred mixture of *tert*-butyl (E)-3-(3-formyl-phenyl)-acrylate (prepared as described in Preparation 1, 372 mg, 1.6 mmol) and KOH (89 mg, 1.6 mmol) in EtOH (10 ml) at -20°C. The resulting mixture was stirred at RT overnight and then partitioned between water and AcOEt. The organic phase was dried over Na₂SO₄ and evaporated *under vacuum.* The crude product was dissolved in DCM (10 ml) and TFA (2 ml) and the resulting solution was stirred at RT for 6 h. The solvent was then removed *under vacuum* and the residue was triturated in Et₂O to give 308 mg of (E)-3-(3-{(E)-3-[3-(4-methyl-piperazin-1-yl)-phenyl]-3-oxo-propenyl}-phenyl)-acrylic acid as its trifluoroacetate salt. The crude compound was used in the next step without any further purifications.

### STEP B

A mixture of (E)-3-(3-{(E)-3-[3-(4-methyl-piperazin-1-yl)-phenyl]-3-oxo-propenyl}-phenyl)-acrylic acid trifluoroacetate (308 mg), HOBT (170 mg, 1.25 mmol), EDC (240 mg, 1.25 mmol), TEA (262 µl, 1.88 mmol) and NH₂OTHP (88 mg, 0.75 mmol) in DMF (7 ml), was stirred overnight at RT and then partitioned between water and AcOEt. The organic extract was dried over Na₂SO₄, evaporated *under vacuum* and the crude reaction mixture was purified by column chromatography (eluent: DCM/MeOH/NH₄OH 98:2:0.2). The collected fractions were evaporated and the resulting product was dissolved in DCM and treated with HCl/Et₂O for 1 h. The precipitate was filtered and purified by preparative LC-MS to give 24.7 mg of (E)-N-hydroxy-3-(3-{(E)-3-[3-(4-methyl-piperazin-1-yl)-phenyl]-3-oxo-propenyl}-phenyl)-acrylamide as its trifluoroacetate salt.
LC-MS: Method C, rt=1.44; (ES+) MH⁺: 392.24
¹H NMR (DMSO-d₆) δ (ppm): 9.74 (bs, 1 H), 8.10 (s, 1 H), 7.96 (d, 1 H), 7.87 (d, 1 H), 7.58 - 7.82 (m, 4 H), 7.40 - 7.58 (m, 3 H), 7.34 (dd, 1 H), 6.57 (d, 1 H), 3.99 (d, 2 H), 3.56 (d, 2 H), 2.97 - 3.30 (m, 4 H), 2.89 (s, 3 H).

### Example 4: (E)-3-(3-{(E)-3-[3-Chloro-5-(4-methyl-piperazin-1-yl)-phenyl]-3-oxo-propenyl}-phenyl)-N-hydroxy-acrylamide

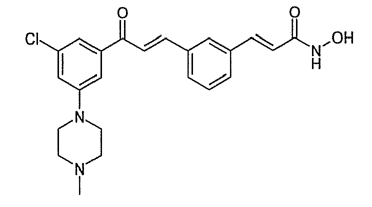

### STEP A

A 1.7 M KOH (0.634 ml) solution was added dropwise to a stirred solution of *tert-*butyl (E)-3-(3-formyl-phenyl)-acrylate (prepared as described in Preparation 1, 250 mg, 1.08 mmol) and 1-[3-chloro-5-(4-methyl-piperazin-1-yl)-phenyl]-ethanone (prepared as described in Preparation 6, 272 mg, 1.08 mmol) in EtOH (15 ml) at 0°C. The resulting mixture was stirred at 0°C for 7 h and then partitioned between water and AcOEt. The organic phase was dried over Na₂SO₄ and evaporated *under vacuum.* The crude mixture was purified by column chromatography (eluent: DCM/MeOH/NH₄OH 98:2:0.2) to give *tert*-butyl (E)-3-(3-{(E)-3-[3-chloro-5-(4-methyl-piperazin-1-yl)-phenyl]-3-oxo-propenyl}-phenyl)-acrylate (0.164 g).
Y=33%

### STEP B

A mixture of *tert*-butyl (E)-3-(3-{(E)-3-[3-chloro-5-(4-methyl-piperazin-1-yl)-phenyl]-3-oxo-propenyl}-phenyl)-acrylate (160 mg, 0.34 mmol) and TFA (1 ml) in DCM (5 ml) was stirred at RT for 18 h. The solvent was removed *under vacuum* and the residue was suspended in DCM (4 ml). HOBT (56 mg, 0.41 mmol), EDC (76 mg, 0.39 mmol), TEA (47 µl, 0.34 mmol) and NH₂OTHP (48 mg, 0.41 mmol) were added and the resulting mixture was stirred at RT overnight. DCM was removed and the residue was purified by two successive column chromatographies (eluent: DCM/MeOH/NH₄OH 98:2:0.2 and then with petroleum ether/AcOEt/TEA from 1:1:0.1 to 1:4:0.2). The resulting product was dissolved in DCM (5 ml) and treated with HCl/Et₂O for 4 h. The precipitate was filtered and rinsed with DCM and Et₂O to give (E)-3-(3-{(E)-3-[3-chloro-5-(4-methyl-piperazin-1-yl)-phenyl]-3-oxo-propenyl}-phenyl)-N-hydroxy-acrylamide hydrochloride (0.047 g).
Y=30 %
LC-MS: Method A, rt=1.58; (ES+) MH⁺: 426.11
¹H NMR (DMSO-d₆ +TFA) δ (ppm): 10.53 (bs, 1 H), 8.07 - 8.22 (m, 1 H), 7.97 (d, 1 H), 7.86 - 7.93 (m, 1 H), 7.73 - 7.86 (m, 1 H), 7.63 - 7.73 (m, 2 H), 7.58 (dd, 1 H), 7.53 (d, 1 H), 7.51 (t, 1 H), 7.38 (t, 1 H), 6.59 (d, 1 H), 3.97 - 4.11 (m, 2 H), 3.39 - 3.65 (m, 2 H), 3.03 - 3.35 (m, 4 H), 2.84 (d, 3 H).

### Example 5: (E)-N-Hydroxy-3-(3-{(E)-3-[3-(4-methyl-piperazin-1-yl-methyl)-phenyl]-3-oxo-propenyl}-phenyl)-acrylamide

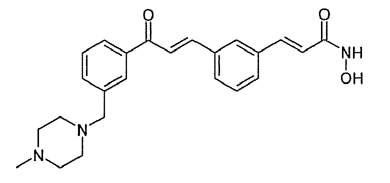

The product was obtained starting from 1-[3-(4-methyl-piperazin-1-yl-methyl)-phenyl]-ethanone (prepared as described in Preparation 4) and *tert*-butyl (E)-3-(3-formyl-phenyl)-acrylate (prepared as described in Preparation 1) following the procedure described at Example 2. The title compound was purified by preparative LC-MS and was obtained as its bis-trifluoroacetate salt.
LC-MS: Method B, rt=1.71; (ES+) MH⁺: 406.24
¹H NMR (DMSO-d₆ 353K +TFA) δ (ppm): 8.12 - 8.18 (m, 1 H), 8.09 (dt, 1 H), 7.98 - 8.05 (m, 1 H), 7.87 (d, 1 H), 7.82 (dt, 1 H), 7.74 (d, 1 H), 7.71 (dt, 1 H), 7.64 (dt, 1 H), 7.59 (t, 1 H), 7.53 (d, 1 H), 7.50 (t, 1 H), 6.67 (d, 1 H), 3.96 (s, 2 H), 3.22 - 3.41 (m, 4 H), 2.88 - 3.05 (m, 4 H), 2.79 (s, 3 H).

### Example 6: (E)-N-Hydroxy-3-(3-{(E)-3-[4-(4-methyl-piperazin-1-yl)-phenyl]-3-oxo-propenyl}-phenyl)-acrylamide

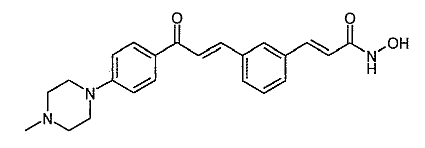

### STEP A

A mixture of *tert*-butyl (E)-3-(3-formyl-phenyl)-acrylate (prepared as described in Preparation 1, 425 mg, 1.83 mmol), 1-[4-(4-methyl-piperazin-1-yl)-phenyl]-ethanone (400 mg, 1.83 mmol) and KOH (202 mg, 3.6 mmol) in EtOH (5 ml) and H₂O (1 ml) was stirred at RT for 6 h. The mixture was diluted with water and the resulting precipitate was filtered. The solid was dissolved in DCM (4 ml) and TFA (2 ml) and the solution was stirred at RT overnight. The solvent was then removed *under vacuum* and the crude product was triturated with *i*-Pr₂O to give (E)-3-(3-{(E)-3-[4-(4-methyl-piperazin-1-yl)-phenyl]-3-oxo-propenyl}-phenyl)-acrylic acid as its trifluoroacetate salt (0.360 g). The crude compound was used in the next step without any further purification.

### STEP B

A mixture of (E)-3-(3-{(E)-3-[4-(4-methyl-piperazin-1-yl)-phenyl]-3-oxo-propenyl}-phenyl)-acrylic acid trifluoroacetate (as obtained in STEP A, 150 mg), HOBT (65 mg, 0.47 mmol), EDC (90 mg, 0.47 mmol), TEA (0.166 ml, 1.19 mmol) and NH₂OTHP (46 mg, 0.40 mmol) in THF (5 ml) and DMF (1 ml), was stirred at RT overnight and then partitioned between water and AcOEt. The organic extract was dried over Na₂SO₄ and evaporated *under vacuum.* The crude product was purified by column chromatography (eluent: AcOEt to AcOEt/MeOH 9:1). The collected fractions gave 70 mg of a yellow powder that was dissolved in DCM and treated with HCl/Et₂O for 2 h. The precipitate was filtered and triturated with MeOH/H₂O to give (E)-N-hydroxy-3-(3-{(E)-3-[4-(4-methyl-piperazin-1-yl)-phenyl]-3-oxo-propenyl}-phenyl)-acrylamide hydrochloride (35 mg).
LC-MS: Method C, rt=1.36; (ES+) MH⁺: 392.31
¹H NMR (DMSO-d₆ + TFA) δ (ppm): 10.36 (bs, 1 H), 8.12 (d, 2 H), 8.10 (bs, 1 H), 8.00 (d, 1 H), 7.85 (d, 1 H), 7.69 (d, 1 H), 7.63 (d, 1 H), 7.44 - 7.58 (m, 2 H), 7.12 (d, 2 H), 6.58 (d, 1 H), 4.14 (d, 2 H), 3.53 (d, 2 H), 3.04 - 3.32 (m, 4 H), 2.85 (s, 3 H).

### Example 7: (E)-N-Hydroxy-3-(3-{(E)-3-[4-(4-methyl-piperazin-1-yl-methyl)-phenyl]-3-oxo-propenyl}-phenyl)-acrylamide

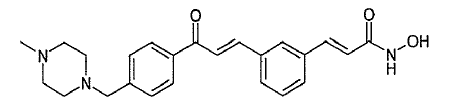

### STEP A

A mixture of *tert*-butyl (E)-3-(3-formyl-phenyl)-acrylate (prepared as described in Preparation 1, 209 mg, 0.90 mmol), 1-[4-(4-methyl-piperazin-1-yl-methyl)-phenyl]-ethanone (prepared as described in Preparation 5, 194 mg, 0.83 mmol) and 1.7 M KOH (0.512 ml) in EtOH (10 ml) was stirred at 0-4° C overnight and then partitioned between water and AcOEt. The organic phase was dried over Na₂SO₄ and evaporated *under vacuum.* The crude mixture was purified by chromatographic column (eluent: DCM/MeOH/NH₄OH 97:3:0.1) to give *tert*-butyl-(E)-3-(3-{(E)-3-[4-(4-methyl-piperazin-1-yl-methyl)-phenyl]-3-oxo-propenyl}-phenyl)-acrylate (0.148 g).
Y=40%

### STEP B

*tert*-Butyl (E)-3-(3-{(E)-3-[4-(4-methyl-piperazin-1-yl-methyl)-phenyl]-3-oxo-propenyl}-phenyl)-acrylate (148 mg, 0.33 mmol) was stirred in a mixture of TFA (0.50 ml) and DCM (1 ml) at RT for 4 h. The solvent was removed *under vacuum* and the residue was dissolved in DCM (3 ml) and DMF (1 ml). HOBT (81 mg, 0.6 mmol), EDC (115 mg, 0.59 mmol), TEA (0.167 ml, 1.2 mmol) and NH₂OTHP (70 mg, 0.6 mmol) were added and the resulting mixture was stirred at RT overnight. DCM was removed and the residue partitioned between water and AcOEt. The organic extract was washed with water, dried over Na₂SO₄ and evaporated *under vacuum.* The crude mixture was purified by column chromatography (eluent: DCM/MeOH/NH₄OH 95:5:0.1) and the resulting product was dissolved in DCM and treated with HCl/Et₂O for 3 h. The precipitate was filtered, crystallized from i-PrOH and then triturated in MeOH to give (E)-N-hydroxy-3-(3-{(E)-3-[4-(4-methyl-piperazin-1-yl-methyl)-phenyl]-3-oxo-propenyl}-phenyl)-acrylamide bis-hydrochloride (54 mg).
Y=34 %
LC-MS: Method A, rt=1.23; (ES+) MH⁺: 406.24
¹H NMR (DMSO-d₆ 353K) δ (ppm): 8.13 (m, 2 H), 7.99 - 8.08 (m, 1 H), 7.84 - 7.93 (m, 1 H), 7.78 - 7.84 (m, 1 H), 7.74 (d, 1 H), 7.69 (m, 2 H), 7.60 - 7.66 (m, 1 H), 7.53 (d, 1 H), 7.50 (t, 1 H), 6.67 (d, 1 H), 4.05 (s, 2 H), 3.22 - 3.49 (m, 4 H), 2.96 - 3.21 (m, 4 H), 2.78 (s, 3 H).

### Example 8: (E)-N-Hydroxy-3-(6-{(E)-3-[2-(4-methyl-piperazin-1-yl)-phenyl]-3-oxo-propenyl}-pyridin-2-yl)-acrylamide

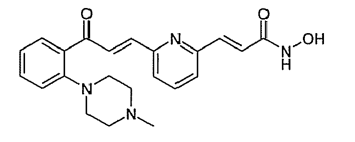

### STEP A

A solution of *tert*-butyl (E)-3-(6-formyl-pyridin-2-yl)-acrylate (prepared as described in Preparation 7, 200 mg, 0.86 mmol), 1-[2-(4-methyl-piperazin-1-yl)-phenyl]-ethanone (prepared as described in Preparation 2, 187 mg, 0.86 mmol) and 1.7 M KOH (0.504 ml) in THF (10 ml) was stirred at RT overnight and then partitioned between water and AcOEt. The aqueous phase was extracted with AcOEt and the collected organic layers were dried over Na₂SO₄ and evaporated *under vacuum.* The crude product was purified by column chromatography (eluent: DCM/MeOH/NH₄OH 97:3:0.2 to 96:4:0.2) to give of *tert*-butyl (E)-3-(6-{(E)-3-[2-(4-methyl-piperazin-1-yl)-phenyl]-3-oxo-propenyl}-pyridin-2-yl)-acrylate (0.240 g).
Y=64%

### STEP B

*tert*-Butyl (E)-3-(6-{(E)-3-[2-(4-methyl-piperazin-1-yl)-phenyl]-3-oxo-propenyl}-pyridin-2-yl)-acrylate (240 mg, 0.55 mmol) was stirred in a mixture of TFA (1 ml) and DCM (4 ml) at RT for 8 h. The solvents were removed *under vacuum* and the residue was dissolved in DMF (15 ml). HOBT (150 mg, 1.11 mmol), EDC (212 mg, 1.11 mmol), and TEA (0.231 ml, 1.66 mmol) were added and the resulting solution was stirred for 10 min. NH₂OTHP (77.8 mg, 0.665 mmol) was added and the mixture was stirred at RT for 7 h. Further NH₂OTHP (7.78 mg, 0.067 mmol) was added and the solution was stirred at RT overnight and then partitioned between water and DCM. The aqueous phase was washed twice with DCM and the collected organic layers were dried over Na₂SO₄ and evaporated *under vacuum.* The crude mixture was purified by column chromatography (eluent: DCM/MeOH/NH₄OH 97:3:0.2 to 95:5:0.2). The resulting product was dissolved in DCM and treated with Et₂O/HCl for 4 h. The precipitate was filtered and freeze dried to give (E)-N-hydroxy-3-(6-{(E)-3-[2-(4-methyl-piperazin-1-yl)-phenyl]-3-oxo-propenyl}-pyridin-2-yl)-acrylamide bis-hydrochloride (40 mg).
Y=15%
LC-MS: Method A, rt=1.30; (ES+) MH⁺: 393.18
¹H NMR (DMSO-d₆) δ (ppm): 10.92 (bs, 1 H), 10.58 (bs, 1 H), 7.96 (d, 1 H), 7.95 (t, 1 H), 7.74 - 7.81 (m, 1 H), 7.47 - 7.70 (m, 5 H), 7.29 - 7.37 (m, 1 H), 7.24 (t, 1 H), 7.04 (d, 1 H), 3.30 - 3.55 (m, 4 H), 3.12 - 3.30 (m, 2 H), 2.82 - 3.01 (m, 2 H), 2.58 (d, 3 H).

### Example 9: (E)-N-Hydroxy-3-(6-{(E)-3-[2-(4-methyl-piperazin-1-yl-methyl)-phenyl]-3-oxo-propenyl}-pyridin-2-yl)-acrylamide

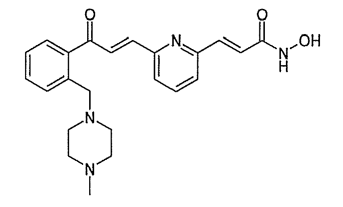

### STEP A

A solution of 1-[2-(4-methyl-piperazin-1-yl-methyl)-phenyl]-ethanone (prepared as described in Preparation 3, 110 mg, 0.47 mmol) in THF (4 ml) was added dropwise within 30 min to a stirred solution of *tert*-butyl (E)-3-(6-formyl-pyridin-2-yl)-acrylate (described in Preparation 7, 110 mg, 0.47 mmol) and 1.7 M KOH (0.276 ml) in THF (10 ml) cooled down to 0°C. The mixture was stirred at 0°C for 1 h and then at RT overnight. The solution was partitioned between water and AcOEt and the organic phase was dried over Na₂SO₄ and evaporated *under vacuum.* The crude mixture was purified by column chromatography (eluent: DCM/MeOH/NH₄OH 98:2:0.1) to give *tert*-butyl (E)-3-(6-{(E)-3-[2-(4-methyl-piperazin-1-yl-methyl)-phenyl]-3-oxo-propenyl}-pyridin-2-yl)-acrylate (0.135 g).
Y=64 %

### STEP B

*tert*-Butyl (E)-3-(6-{(E)-3-[2-(4-methyl-piperazin-1-yl-methyl)-phenyl]-3-oxo-propenyl}-pyridin-2-yl)-acrylate (135 mg, 0.30 mmol) was stirred in a mixture of TFA (0.456 ml) and DCM (1 ml) at RT for 4 h. The solvents were evaporated to dryness and the residue was dissolved in DCM (4 ml) and TEA (0.581 ml, 4.18 mmol). EDC (145 mg, 0.756 mmol), HOBT (103 mg, 0.76 mmol), and NH₂OTHP (66.7 mg, 0.570 mmol) were added and the resulting mixture was stirred at RT overnight. The solvent was evaporated and the residue was partitioned between water and AcOEt. The organic phase was dried over Na₂SO₄ and evaporated *under vacuum.* The crude mixture was purified by column chromatography (eluent: DCM:MeOH:NH₄OH 93:7:0.1) and the resulting product was dissolved in DCM and treated with HCl/Et₂O for 1.5 h. The precipitate was filtered, triturated with *i*-PrOH and purified by preparative LC-MS to give (E)-N-hydroxy-3-(6-{(E)-3-[2-(4-methyl-piperazin-1-yl-methyl)-phenyl]-3-oxo-propenyl}-pyridin-2-yl)-acrylamide tris-trifluoroacetate (36 mg).
Y=16%
LC-MS: Method A, rt=1.44; (ES+) MH⁺: 407.20
¹H NMR (DMSO-d₆ +TFA) δ (ppm): 10.92 (bs, 1 H), 9.47 (bs, 1 H), 7.92 (t, 1 H), 7.79 (d, 1 H), 7.41 - 7.68 (m, 7 H), 7.30 (d, 1 H), 7.00 (d, 1 H), 3.75 (bs, 2 H), 3.24 - 3.40 (m, 2 H), 2.73 - 3.00 (m, 4 H), 2.71 (s, 3 H), 2.30 - 2.45 (m, 2 H).

### Example 10: (E)-N-Hydroxy-3-(6-{(E)-3-[3-(4-methyl-piperazin-1-yl)-phenyl]-3-oxo-propenyl}-pyridin-2-yl)-acrylamide

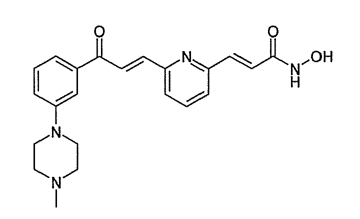

A solution of 1-[3-(4-methyl-piperazin-1-yl)-phenyl]-ethanone (137 mg, 0.63 mmol) in THF (10 ml) was added dropwise within 30 min to a stirred solution of *tert*-butyl (E)-3-(6-formyl-pyridin-2-yl)-acrylate (described in Preparation 7, 146 mg, 0.63 mmol) and 1.7 M KOH (370 µl) in THF (10 ml) at 0°C. The mixture was stirred at 0°C for 1 h and then at RT overnight. The solution was partitioned between water and AcOEt and the organic phase was dried over Na₂SO₄ and evaporated *under vacuum.* The crude product was purified by column chromatography (eluent: DCM/MeOH/NH₄OH 98:2:0.1) to give *tert*-butyl (E)-3-(6-{(E)-3-[3-(4-methyl-piperazin-1-yl)-phenyl]-3-oxo-propenyl}-pyridin-2-yl)-acrylate (0.188 g) as an orange solid.
Y=69 %

### STEP B

*tert*-Butyl (E)-3-(6-{(E)-3-[3-(4-methyl-piperazin-1-yl)-phenyl]-3-oxo-propenyl}-pyridin-2-yl)-acrylate (188 mg, 0.434 mmol) was stirred in a mixture of TFA (1.5 ml) and DCM (3 ml) at RT for 2 h. The solvents were evaporated to dryness and the residue was dissolved in DCM (4 ml) and TEA (0.800 ml, 5.80 mmol). EDC (198 mg, 1.03 mmol), HOBT (140 mg, 1.04 mmol) and NH₂OTHP (100 mg, 0.854 mmol) were added and the resulting mixture was stirred at RT overnight. The solvent was evaporated and the residue was partitioned between water and AcOEt. The organic phase was dried over Na₂SO₄ and evaporated *under vacuum.* The crude product was purified by column chromatography (eluent: DCM:MeOH:NH₄OH 97:3:0.1) and the resulting product was dissolved in DCM and treated with HCl/Et₂O for 2 h. The precipitate was filtered, triturated with i-PrOH and purified by preparative LC-MS to give 27 mg of (E)-N-hydroxy-3-(6-{(E)-3-[3-(4-methyl-piperazin-1-yl)-phenyl]-3-oxo-propenyl}-pyridin-2-yl)-acrylamide bis-trifluoroacetate salt.
Y=10%
LC-MS: Method A, rt=1.22; (ES+) MH⁺: 393.25
¹H NMR (DMSO-d₆) δ (ppm): 10.94 (bs, 1 H), 9.80 (bs, 1 H), 8.12 (d, 1 H), 7.96 (t, 1 H), 7.91 (dd, 1 H), 7.71 (d, 1 H), 7.55 - 7.67 (m, 3 H), 7.51 (d, 1 H), 7.51 (d, 1 H), 7.37 (dd, 1 H), 7.06 (d, 1 H), 3.90 - 4.12 (m, 2 H), 3.43 - 3.64 (m, 2 H), 3.14 - 3.33 (m, 2 H), 2.98 - 3.12 (m, 2 H), 2.89 (s, 3 H).

### Example 11: (E)-3-(6-{(E)-3-[3-Chloro-5-(4-methyl-piperazin-1-yl)-phenyl]-3-oxo-propenyl}-pyridin-2-yl)-N-hydroxy-acrylamide

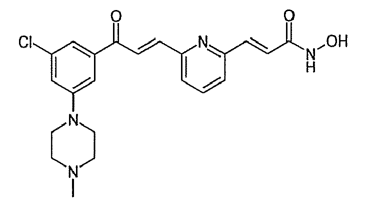

### STEP A

A mixture of 1-[3-chloro-5-(4-methyl-piperazin-1-yl)-phenyl]-ethanone hydrochloride (described in Preparation 6, 216 mg, 0.75 mmol), *tert*-butyl (E)-3-(6-formyl-pyridin-2-yl)-acrylate (described in Preparation 7, 174 mg, 0.75 mmol) and 1.7 M KOH (0.88 ml) in THF (10 ml) was stirred at RT overnight and then partitioned between water and AcOEt. The organic phase was dried over Na₂SO₄ and evaporated to dryness. The crude reaction mixture was purified by column chromatography (eluent: DCM/MeOH/NH₄OH 98:2:0.2) to give *tert*-butyl (E)-3-(6-{(E)-3-[3-chloro-5-(4-methyl-piperazin-1-yl)-phenyl]-3-oxo-propenyl}-pyridin-2-yl)-acrylate (0.193 g) as brown oil.
Y=55 %

### STEP B

*tert*-Butyl (E)-3-(6-{(E)-3-[3-chloro-5-(4-methyl-piperazin-1-yl)-phenyl]-3-oxo-propenyl}-pyridin-2-yl)-acrylate (193 mg, 0.413 mmol) was stirred in a mixture of TFA (0.954 ml) and DCM (5 ml) at RT overnight. The solvents were removed *under vacuum* and the residue was dissolved in DMF (5 ml). EDC (216 mg, 1.13 mmol), HOBT·H₂O (170 mg, 1.13 mmol), TEA (236 µl, 1.70 mmol) and NH₂OTHP (79.5 mg, 0.678 mmol) were added and the resulting solution was stirred at RT overnight. The mixture was diluted with water and extracted with AcOEt and DCM. The collected organic phases were dried over Na₂SO₄ and evaporated *under vacuum.* The crude product was purified by column chromatography (eluent: DCM/MeOH/NH₄OH 97:3:0.2) and the resulting product was dissolved in DCM and treated with HCl/Et₂O for 2 h. The precipitate was filtered and rinsed with DCM and Et₂O to give (E)-3-(6-{(E)-3-[3-chloro-5-(4-methyl-piperazin-1-yl)-phenyl]-3-oxo-propenyl}-pyridin-2-yl)-N-hydroxy-acrylamide as its bis-hydrochloride salt (0.134 g).
Y=65%
LC-MS: Method B, rt=2.12; (ES+) MH⁺: 427.18
¹H NMR (DMSO-d₆) δ (ppm): 11.26 (bs, 1 H), 8.11 (d, 1 H), 7.93 - 8.06 (m, 2 H), 7.64 - 7.79 (m, 2 H), 7.50 - 7.61 (m, 3 H), 7.39 (t, 1 H), 7.10 (d, 1 H), 3.90 - 4.18 (m, 2 H), 3.37 - 3.57 (m, 2 H), 3.29 (t, 2 H), 3.03 - 3.23 (m, 2 H), 2.80 (d, 3 H).

### Example 12: (E)-N-Hydroxy-3-(6-{(E)-3-[3-(4-methyl-piperazin-1-yl-methyl)-phenyl]-3-oxo-propenyl}-pyridin-2-yl)-acrylamide

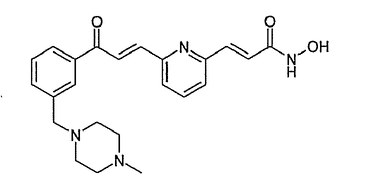

### STEP A

A mixture of 1-[3-(4-methyl-piperazin-1-yl-methyl)-phenyl]-ethanone (prepared as described in Preparation 4, 200 mg, 0.85 mmol), *tert*-butyl (E)-3-(6-formyl-pyridin-2-yl)-acrylate (described in Preparation 7, 200 mg, 0.85 mmol) and 1.7 M KOH (0.504 ml) in THF (10 ml) was stirred at RT overnight and then partitioned between water and AcOEt. The organic phase was dried over Na₂SO₄ and evaporated *under vacuum.* The crude mixture was purified by column chromatography (eluent: DCM/MeOH/NH₄OH 97:3:0.2) to give *tert*-butyl (E)-3-(6-{(E)-3-[3-(4-methyl-piperazin-1-yl-methyl)-phenyl]-3-oxo-propenyl}-pyridin-2-yl)-acrylate (0.300 g).
Y=78%

### STEP B

*tert*-Butyl (E)-3-(6-{(E)-3-[3-(4-methyl-piperazin-1-yl-methyl)-phenyl]-3-oxo-propenyl}-pyridin-2-yl)-acrylate (300 mg, 0.671 mmol) was stirred in a mixture of TFA (1 ml) and DCM (4 ml) at RT for 5 h. Further TFA (1 ml) was added and the mixture was stirred for 4 h. The solvents were removed *under vacuum* and the residue was dissolved in DMF (15 ml). EDC (256 mg, 1.33 mmol), HOBT (181 mg, 1.34 mmol) and TEA (0.280 ml, 2.01 mmol) were added. After 10 min NH₂OTHP (94.1 mg, 0.804 mmol) was added and the resulting mixture was stirred at RT. Further NH₂OTHP (63 mg, 0.54 mmol) was added in two portions over 24 h and then the resulting solution was partitioned between water and DCM. The aqueous phase was extracted three times with DCM, the collected organic layers were dried over Na₂SO₄, and evaporated *under vacuum.* The crude mixture was purified by column chromatography (eluent: DCM:MeOH:NH₄OH from 97:3:0.2 to 96:4:0.2) and the resulting product was dissolved in DCM and treated with HCl/Et₂O for 4 h. The precipitate was filtered, rinsed with DCM and purified by preparative LC-MS to give (E)-N-hydroxy-3-(6-{(E)-3-[3-(4-methyl-piperazin-1-yl-methyl)-phenyl]-3-oxo-propenyl}-pyridin-2-yl)-acrylamide as its tris-trifluoroacetate salt (30 mg).
Y=6%
LC-MS: Method B, rt=1.51; (ES+) MH⁺: 407.21
¹H NMR (DMSO-d₆ +TFA) δ (ppm): 8.15 (d, 1 H), 8.06 - 8.14 (m, 2 H), 7.96 (t, 1 H), 7.83 - 7.93 (m, 1 H), 7.74 (d, 1 H), 7.60 - 7.74 (m, 3 H), 7.55 (d, 1 H), 7.05 (d, 1 H), 3.95 (s, 2 H), 3.05 - 3.99 (m, 8 H), 2.81 (s, 3 H).

### Example 13: (E)-N-Hydroxy-3-(6-{(E)-3-[4-(4-methyl-piperazin-1-yl)-phenyl]-3-oxo-propenyl}-pyridin-2-yl)-acrylamide

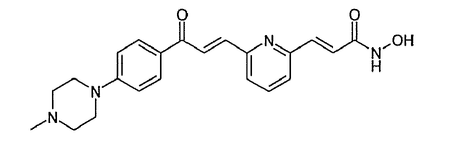

### STEP A

1.7 M KOH (0.580 ml) was added dropwise to a stirred mixture of 1-[4-(4-methyl-piperazin-1-yl)-phenyl]-ethanone (215 mg, 0.987 mmol) and *tert*-butyl (E)-3-(6-formyl-pyridin-2-yl)-acrylate (prepared as described in Preparation 7, 229 mg, 0.987 mmol) in 15 ml of EtOH at 0°C. The resulting solution was stirred at 0°C for 8 h. The slurry was diluted with water and extracted with AcOEt. The organic phase was dried over Na₂SO₄ and evaporated to dryness. The crude product was purified by column chromatography (eluent: DCM/MeOH/NH₄OH 10:4:0.2) to give a mixture (0.34 g) of *tert*-butyl (E)-3-(6-{1-hydroxy-3-[4-(4-methyl-piperazin-1-yl)-phenyl]-3-oxo-propyl}-pyridin-2-yl)-acrylate and *tert*-butyl (E)-3-(6-{(E)-3-[4-(4-methyl-piperazin-1-yl)-phenyl]-3-oxo-propenyl}-pyridin-2-yl)-acrylate. The mixture was dissolved in DCM (10 ml) and TFA (2 ml) and the resulting solution was stirred at RT overnight. The solvent was removed *under vacuum* and the residue was dissolved in THF (10 ml) and 1.7 M KOH (2.64 ml). The solution was stirred at RT for 1 h and then acidified with HCl/Et₂O and evaporated *under vacuum.* The resulting solid was washed with water and filtered to give (E)-3-(6-{(E)-3-[4-(4-methyl-piperazin-1-yl)-phenyl]-3-oxo-propenyl}-pyridin-2-yl)-acrylic acid bis-hydrochloride (0.14 g). The crude product was used in the next step without any further purification.

### STEP B

A mixture of (E)-3-(6-{(E)-3-[4-(4-methyl-piperazin-1-yl)-phenyl]-3-oxo-propenyl}-pyridin-2-yl)-acrylic acid bis-hydrochloride (as obtained in STEP A, 140 mg), EDC (118 mg, 0.62 mmol) HOBT·H₂O (100 mg, 0.62 mmol), TEA (0.258 ml, 1.86 mmol) and NH₂OTHP (44 mg, 0.37 mmol) in DMF (5 ml) was stirred at RT for 6 h.

The resulting solution was diluted with water and extracted with AcOEt and DCM. The collected organic phases were dried over Na₂SO₄ and evaporated *under vacuum*. The resulting crude product was purified by column chromatography (eluent: DCM/MeOH/NH₄OH 96:4:0.2). The product was dissolved in DCM and treated with HCl/Et₂O for 2 h. The precipitate was filtered and rinsed with DCM and Et₂O to give (E)-N-hydroxy-3-(6-{(E)-3-[4-(4-methyl-piperazin-1-yl)-phenyl]-3-oxo-propenyl}-pyridin-2-yl)-acrylamide bis-hydrochloride (68 mg).
LC-MS: Method A, rt=1.13; (ES+) MH⁺: 393.25
¹H NMR (DMSO-d₆ 353K +TFA) δ (ppm): 7.99 - 8.15 (m, 3 H), 7.90 (t, 1 H), 7.74 - 7.81 (m, 1 H), 7.63 (d, 1 H), 7.58 (d, 1 H), 7.52 (d, 1 H), 7.02 - 7.18 (m, 3 H), 3.75 (bs, 4 H), 3.34 (bs, 4 H), 2.84 (s, 3 H).

### Example 14: (E)-N-Hydroxy-3-(6-{(E)-3-[4-(4-methyl-piperazin-1-yl-methyl)-phenyl]-3-oxo-propenyl}-pyridin-2-yl)-acrylamide

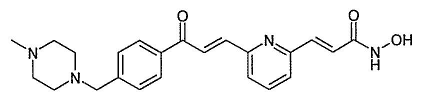

### STEP A

A solution of 1-[4-(4-methyl-piperazin-1-yl-methyl)-phenyl]-ethanone (prepared as described in Preparation 5, 189 mg, 0.815 mmol) in THF (8 ml) was added dropwise within 30 min to a stirred solution of *tert*-butyl (E)-3-(6-formyl-pyridin-2-yl)-acrylate (prepared as described in Preparation 7, 190 mg, 0.815 mmol) and 1.7 M KOH (0.48 ml) in THF (20 ml) at 0°C. The mixture was stirred at 0°C for 1 h and then at RT overnight. The solution was partitioned between water and AcOEt and the organic phase was dried over Na₂SO₄ and evaporated *under vacuum.* The crude product was purified by column chromatography (eluent: DCM/MeOH/NH₄OH 96:4:0.1) to give *tert*-butyl (E)-3-(6-{(E)-3-[4-(4-methyl-piperazin-1-yl-methyl)-phenyl]-3-oxo-propenyl}-pyridin-2-yl)-acrylate (0.245 g)
Y=67 %

### STEP B

*tert*-Butyl (E)-3-(6-{(E)-3-[4-(4-methyl-piperazin-1-yl-methyl)-phenyl]-3-oxo-propenyl}-pyridin-2-yl)-acrylate (245 mg, 0.548 mmol) was stirred in a mixture of TFA (3 ml) and DCM (10 ml) at RT for 2 h. The solvent was evaporated and the residue was dissolved in DCM (6 ml) and TEA (1.1 ml, 8.14 mmol). EDC (283 mg, 1.47 mmol), HOBT (200 mg, 1.48 mmol), and NH₂OTHP (86.6 mg, 0.74 mmol) were added and the resulting mixture was stirred at RT overnight. The solvent was evaporated and the residue was partitioned between water and AcOEt. The organic phase was dried over Na₂SO₄ and evaporated *under vacuum.* The crude product was purified by column chromatography (eluent: DCM:MeOH:NH₄OH 96:4:0.1) and the resulting product was dissolved in DCM and treated with HCl/Et₂O for 3 h. The precipitate was filtered, rinsed with DCM, and purified by preparative LC-MS to give (E)-N-hydroxy-3-(6-{(E)-3-[4-(4-methyl-piperazin-1-yl-methyl)-phenyl]-3-oxo-propenyl}-pyridin-2-yl)-acrylamide as its tris-trifluoroacetate salt (14 mg).
Y=4%
LC-MS: Method A, rt=1.07; (ES+) MH⁺: 407.26
¹H NMR (DMSO-d₆ +TFA) δ (ppm): 8.16 (d, 1 H), 8.13 (m, 2 H), 7.85 - 8.00 (m, 2 H), 7.72 (d, 1 H), 7.48 - 7.68 (m, 4 H), 7.06 (d, 1 H), 3.86 (s, 2 H), 2.88 - 3.55 (m, 8 H), 2.81 (s, 3 H).

The following compounds can be prepared in an analogous manner:
(E)-3-(3-{(E)-3-[2-(4-Acetyl-piperazin-1-yl)-phenyl]-3-oxo-propenyl}-phenyl)-N-hydroxy-acrylamide;
(E)-3-(3-{(E)-3-[3-(4-Acetyl-piperazin-1-yl)-phenyl]-3-oxo-propenyl}-phenyl)-N-hydroxy-acrylamide;
(E)-3-(3-{(E)-3-[4-(4-Acetyl-piperazin-1-yl)-phenyl]-3-oxo-propenyl}-phenyl)-N-hydroxy-acrylamide;
(E)-3-(6-{(E)-3-[2-(4-Acetyl-piperazin-1-yl)-phenyl]-3-oxo-propenyl}-pyridin-2-yl)-N-hydroxy-acrylamide;
(E)-3-(6-{(E)-3-[3-(4-Acetyl-piperazin-1-yl)-phenyl]-3-oxo-propenyl}-pyridin-2-yl)-N-hydroxy-acrylamide;
(E)-3-(6-{(E)-3-[4-(4-Acetyl-piperazin-1-yl)-phenyl]-3-oxo-propenyl}-pyridin-2-yl)-N-hydroxy-acrylamide;
(E)-3-(3-{(E)-3-[2-(4-Acetyl-piperazin-1-ylmethyl)-phenyl]-3-oxo-propenyl}-phenyl)-N-hydroxy-acrylamide;
(E)-3-(3-{(E)-3-[3-(4-Acetyl-piperazin-1-ylmethyl)-phenyl]-3-oxo-propenyl}-phenyl)-N-hydroxy-acrylamide;
(E)-3-(3-{(E)-3-[4-(4-Acetyl-piperazin-1-ylmethyl)-phenyl]-3-oxo-propenyl}-phenyl)-N-hydroxy-acrylamide;
(E)-3-(6-{(E)-3-[2-(4-Acetyl-piperazin-1-ylmethyl)-phenyl]-3-oxo-propenyl}-pyridin-2-yl)-N-hydroxy-acrylamide;
(E)-3-(6-{(E)-3-[3-(4-Acetyl-piperazin-1-ylmethyl)-phenyl]-3-oxo-propenyl}-pyridin-2-yl)-N-hydroxy-acrylamide;
(E)-3-(6-{(E)-3-[4-(4-Acetyl-piperazin-1-ylmethyl)-phenyl]-3-oxo-propenyl}-pyridin-2-yl)-N-hydroxy-acrylamide;
(E)-3-(3-{(E)-3-[2-(4-Phenyl-piperazin-1-ylmethyl)-phenyl]-3-oxo-propenyl}-phenyl)-N-hydroxy-acrylamide;
(E)-3-(3-{(E)-3-[3-(4-Phenyl-piperazin-1-ylmethyl)-phenyl]-3-oxo-propenyl}-phenyl)-N-hydroxy-acrylamide;
(E)-3-(3-{(E)-3-[4-(4-Phenyl-piperazin-1-ylmethyl)-phenyl]-3-oxo-propenyl}-phenyl)-N-hydroxy-acrylamide;
(E)-3-(6-{(E)-3-[2-(4-Phenyl-piperazin-1-ylmethyl)-phenyl]-3-oxo-propenyl}-pyridin-2-yl)-N-hydroxy-acrylamide;
(E)-3-(6-{(E)-3-[3-(4-Phenyl-piperazin-1-ylmethyl)-phenyl]-3-oxo-propenyl}-pyridin-2-yl)-N-hydroxy-acrylamide;
(E)-3-(6-{(E)-3-[4-(4-Phenyl-piperazin-1-ylmethyl)-phenyl]-3-oxo-propenyl}-pyridin-2-yl)-N-hydroxy-acrylamide;
(E)-3-(3-{(E)-3-[2-(4-Benzyl-piperazin-1-yl)-phenyl]-3-oxo-propenyl}-phenyl)-N-hydroxy-acrylamide;
(E)-3-(3-{(E)-3-[3-(4-Benzyl-piperazin-1-yl)-phenyl]-3-oxo-propenyl}-phenyl)-N-hydroxy-acrylamide;
(E)-3-(3-{(E)-3-[4-(4-Benzyl-piperazin-1-yl)-phenyl]-3-oxo-propenyl}-phenyl)-N-hydroxy-acrylamide;
(E)-3-(6-{(E)-3-[2-(4-Benzyl-piperazin-1-yl)-phenyl]-3-oxo-propenyl}-pyridin-2-yl)-N-hydroxy-acrylamide;
(E)-3-(6-{(E)-3-[3-(4-Benzyl-piperazin-1-yl)-phenyl]-3-oxo-propenyl}-pyridin-2-yl)-N-hydroxy-acrylamide;
(E)-3-(6-{(E)-3-[4-(4-Benzyl-piperazin-1-yl)-phenyl]-3-oxo-propenyl}-pyridin-2-yl)-N-hydroxy-acrylamide;
(E)-3-(3-{(E)-3-[2-(4-Benzyl-piperazin-1-ylmethyl)-phenyl]-3-oxo-propenyl}-phenyl)-N-hydroxy-acrylamide;
(E)-3-(3-{(E)-3-[3-(4-Benzyl-piperazin-1-ylmethyl)-phenyl]-3-oxo-propenyl}-phenyl)-N-hydroxy-acrylamide;
(E)-3-(3-{(E)-3-[4-(4-Benzyl-piperazin-1-ylmethyl)-phenyl]-3-oxo-propenyl}-phenyl)-N-hydroxy-acrylamide;
(E)-3-(6-{(E)-3-[2-(4-Benzyl-piperazin-1-ylmethyl)-phenyl]-3-oxo-propenyl}-pyridin-2-yl)-N-hydroxy-acrylamide;
(E)-3-(6-{(E)-3-[3-(4-Benzyl-piperazin-1-ylmethyl)-phenyl]-3-oxo-propenyl}-pyridin-2-yl)-N-hydroxy-acrylamide;
(E)-3-(6-{(E)-3-[4-(4-Benzyl-piperazin-1-ylmethyl)-phenyl]-3-oxo-propenyl}-pyridin-2-yl)-N-hydroxy-acrylamide;
(E)-N-Hydroxy-3-(3-{(E)-3-oxo-3-[2-((3R,5S)-3,4,5-trimethyl-piperazin-1-yl)-phenyl]-propenyl}-phenyl)-acrylamide;
(E)-N-Hydroxy-3-(3-{(E)-3-oxo-3-[3-((3R,5S)-3,4,5-trimethyl-piperazin-1-yl)-phenyl]-propenyl}-phenyl)-acrylamide;
(E)-N-Hydroxy-3-(3-{(E)-3-oxo-3-[4-((3R,5S)-3,4,5-trimethyl-piperazin-1-yl)-phenyl]-propenyl}-phenyl)-acrylamide;
(E)-N-Hydroxy-3-(6-{(E)-3-oxo-3-[2-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-yl)-phenyl]-propenyl}-pyridin-2-yl)-acrylamide;
(E)-N-Hydroxy-3-(3-{(E)-3-oxo-3-[2-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[3-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[4-((3R,5S)-3,4,5-trimethyl-piperazin-1-ylmethyl)-phenyl]-propenyl}-phenyl)-acrylamide;
(E)-N-Hydroxy-3-(6-{(E)-3-oxo-3-[2-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-ylmethyl)-phenyl]-propenyl}-pyridin-2-yl)-acrylamide;
(E)-3-(3-{(E)-3-[2-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-yl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-yl)-phenyl]-3-oxo-propenyl}-pyridin-2-yl)-N-hydroxy-acrylamide;
(E)-3-(3-{(E)-3-[2-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-ylmethyl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-N-Hydroxy-3-{3-[(E)-3-(2-morpholin-4-yl-phenyl)-3-oxo-propenyl]-phenyl}-acrylamide;
(E)-N-Hydroxy-3-{3-[(E)-3-(3-morpholin-4-yl-phenyl)-3-oxo-propenyl]-phenyl}-acrylamide;
(E)-N-Hydroxy-3-{3-[(E)-3-(4-morpholin-4-yl-phenyl)-3-oxo-propenyl]-phenyl}-acrylamide;
(E)-N-Hydroxy-3-{6-[(E)-3-(2-morpholin-4-yl-phenyl)-3-oxo-propenyl]-pyridin-2-yl}-acrylamide;
(E)-N-Hydroxy-3-{6-[(E)-3-(3-morpholin-4-yl-phenyl)-3-oxo-propenyl]-pyridin-2-yl}-acrylamide;
(E)-N-Hydroxy-3-{6-[(E)-3-(4-morpholin-4-yl-phenyl)-3-oxo-propenyl]-pyridin-2-yl}-acrylamide;
(E)-N-Hydroxy-3-{3-[(E)-3-(2-morpholin-4-ylmethyl-phenyl)-3-oxo-propenyl]-phenyl}-acrylamide;
(E)-N-Hydroxy-3-{3-[(E)-3-(3-morpholin-4-ylmethyl-phenyl)-3-oxo-propenyl]-phenyl}-acrylamide;
(E)-N-Hydroxy-3-{3-[(E)-3-(4-morpholin-4-ylmethyl-phenyl)-3-oxo-propenyl]-phenyl}-acrylamide;
(E)-N-Hydroxy-3-{6-[(E)-3-(2-morpholin-4-ylmethyl-phenyl)-3-oxo-propenyl]-pyridin-2-yl}-acrylamide;
(E)-N-Hydroxy-3-{6-[(E)-3-(3-morpholin-4-ylmethyl-phenyl)-3-oxo-propenyl]-pyridin-2-yl}-acrylamide;
(E)-N-Hydroxy-3-{6-[(E)-3-(4-morpholin-4-ylmethyl-phenyl)-3-oxo-propenyl]-pyridin-2-yl}-acrylamide.

Alternatively, the following compounds can be prepared according to the above described procedures starting from the N-Boc protected piperazine derivatives. The Boc group is cleaved in the final step together with the THP group in HCl/ether at the same conditions as described above:
(E)-N-Hydroxy-3-{3-[(E)-3-oxo-3-(2-piperazin-1-yl-phenyl)-propenyl]-phenyl}-acrylamide;
(E)-N-Hydroxy-3-{3-[(E)-3-oxo-3-(3-piperazin-1-yl-phenyl)-propenyl]-phenyl}-acrylamide;
(E)-N-Hydroxy-3-{3-[(E)-3-oxo-3-(4-piperazin-1-yl-phenyl)-propenyl]-phenyl}-acrylamide;
(E)-N-Hydroxy-3-{6-[(E)-3-oxo-3-(2-piperazin-1-yl-phenyl)-propenyl]-pyridin-2-yl}-acrylamide;
(E)-N-Hydroxy-3-{6-[(E)-3-oxo-3-(3-piperazin-1-yl-phenyl)-propenyl]-pyridin-2-yl}-acrylamide;
(E)-N-Hydroxy-3-{6-[(E)-3-oxo-3-(4-piperazin-1-yl-phenyl)-propenyl]-pyridin-2-yl}-acrylamide;
(E)-N-Hydroxy-3-{3-[(E)-3-oxo-3-(2-piperazin-1-ylmethyl-phenyl)-propenyl]-phenyl}-acrylamide;
(E)-N-Hydroxy-3-{3-[(E)-3-oxo-3-(3-piperazin-1-ylmethyl-phenyl)-propenyl]-phenyl}-acrylamide;
(E)-N-Hydroxy-3-{3-[(E)-3-oxo-3-(4-piperazin-1-ylmethyl-phenyl)-propenyl]-phenyl}-acrylamide;
(E)-N-Hydroxy-3-{6-[(E)-3-oxo-3-(2-piperazin-1-ylmethyl-phenyl)-propenyl]-pyridin-2-yl}-acrylamide;
(E)-N-Hydroxy-3-{6-[(E)-3-oxo-3-(3-piperazin-1-ylmethyl-phenyl)-propenyl]-pyridin-2-yl}-acrylamide;
(E)-N-Hydroxy-3-{6-[(E)-3-oxo-3-(4-piperazin-1-ylmethyl-phenyl)-propenyl]-pyridin-2-yl}-acrylamide;
(E)-3-(3-{(E)-3-[2-((3R,5S)-3,5-Dimethyl-piperazin-1-yl)-phenyl]-3-oxo-propenyl}-phenyl)-N-hydroxy-acrylamide;
(E)-3-(3-{(E)-3-[3-((3R,5S)-3,5-Dimethyl-piperazin-1-yl)-phenyl]-3-oxo-propenyl}-phenyl)-N-hydroxy-acrylamide;
(E)-3-(3-{(E)-3-[4-((3R,5S)-3,5-Dimethyl-piperazin-1-yl)-phenyl]-3-oxo-propenyl}-phenyl)-N-hydroxy-acrylamide;
(E)-3-(6-{(E)-3-[2-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-yl)-phenyl]-3-oxo-propenyl}-pyridin-2-yl)-N-hydroxy-acrylamide;
(E)-3-(3-{(E)-3-[2-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[3-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[4-((3R,5S)-3,5-Dimethyl-piperazin-1-ylmethyl)-phenyl]-3-oxo-propenyl}-phenyl)-N-hydroxy-acrylamide;
(E)-3-(6-{(E)-3-[2-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-ylmethyl)-phenyl]-3-oxo-propenyl}-pyridin-2-yl)-N-hydroxy-acrylamide.

### 2. BIOLOGICAL TESTING

### Methods and results

### 2.1 Histone acetylation assay

In order to assess the ability of the compounds to modify histone acetylation levels, a dose-response study was carried out using the cell line K562 (derived from human lymphoma). Briefly, the cells were incubated with the compound for 3 h, then fixed with 1% formaldehyde in PBS and permeabilized with a solution containing 0.1 % Triton X-100 in PBS. After washing, the cells were pre-incubated with 10% goat serum in PBS for 30 min at 4°C, exposed for 1 h at RT to a monoclonal antibody against acetylated histones and then incubated for 1 h with a secondary antibody conjugated with FITC. Histone acetylation levels were measured by cytofluorometry (FACS) (Ronzoni, S. et al. Cytometry A. 2005, 66, 52-61).

The compounds of the present invention showed a remarkable histone deacetylase inhibitory activity (calculated on increase in acetylation) at low nanomolar concentrations.

### 2.2 Assay of enzyme inhibition of HDAC

The in-vitro activity of HDAC inhibitors was assayed by means of a biochemical assay using a BIOMOL Kit, according to the instructions from the manufacturer (Biomolecular Research Laborator). Briefly, 15 µl of 30 x diluted nuclear fraction of Hela cells, was diluted to 50 µl with the assay buffer containing the HDAC inhibitor and the substrate (lysine with acetylated amino on the side chain) at a concentration of 200 µM. The samples were incubated for 15 min at RT and then exposed to a developer (10 min at RT). In this last step a fluorophore was produced, whose fluorescence was measured using an excitation wavelength of 355 nm and an emission at 460 nm. The IC₅₀ is calculated using GraphPad Software.

The obtained results are illustrated in the following table. The reference HDAC inhibitors marked with (*) are those disclosed in the patent application WO2006/037761.

As evident from comparison with the reference HDAC inhibitors of the prior art, the compounds of the invention showed a significant enhancement of activity.

**Table:**

| **Example Nbr** | **Structure** | **Activity IC₅₀[µM]** |
|---|---|---|
| **3** | 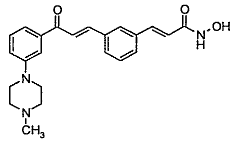 | **0.020** |
| **5** | 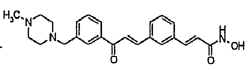 | **0.024** |
| **6** | 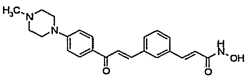 | **0.027** |
| **7** | 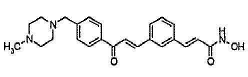 | **0.017** |
| **8** | 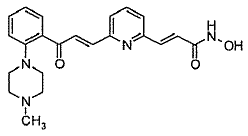 | **0.033** |
| **9** | 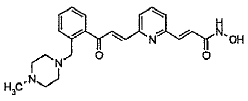 | **0.053** |
| **10** | 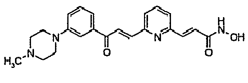 | **0.032** |
| **11** | 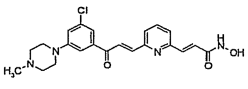 | **0.029** |
| **12** | 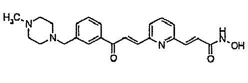 | **0.056** |
| **13** | 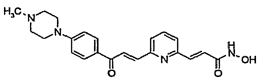 | **0.0145** |
| **14** | 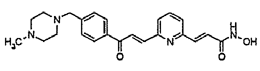 | **0.0407** |
| **Ref (*)MC1646** | 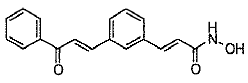 | **0.0853** |
| **Ref (*)MC1653** | 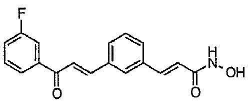 | **1.3749** |
| **Ref (*)MC1661** | 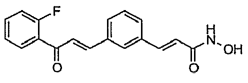 | **0.1302** |
| **Ref (*)MC1670** | 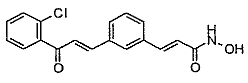 | **0.3892** |
| **Ref (*)MC1672** | 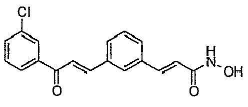 | **1.457** |
| **Ref (*) 59** | 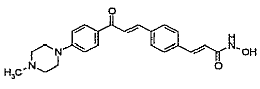 | **1.995** |
| **Ref (*) 41** | 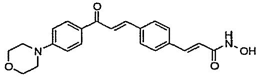 | **1.3598** |
| **Ref (*) 29** | 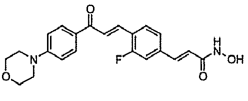 | **2.8404** |
| **Ref (*) 48** | 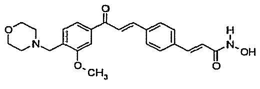 | **0.0733** |

### 2.3 Cell growth: MTT assay

The MTT [3-(4,5-dimethylthiazolyl)-2,5-diphenyltetrazolium bromide] test is a colorimetric test able to measure cell viability and proliferation, based on the capacity of cells to metabolise tetrazolium salt to form formazan crystals by means of a mitochondrial dehydrogenase. The cells in exponential growth phase are exposed to the inhibitors. The activity of the mitochondrial dehydrogenase and the quantity of formazan salts produced are proportional to the number of survived cells. The amount of formazan produced is followed by UV-vis spectrophotometry. K562 cells were incubated for 72 h with different concentrations of the inhibitors. 5 mg/ml of MTT in PBS were added at different time points and the solution was incubated for 3-4 h at 37°C. The supernatant was then removed and the formazan crystals were dissolved in a mixture of DMSO and absolute EtOH (1:1, v:v) and the solution analysed with a spectrophotometer at a wavelength between 550 and 570 nm. The IC₅₀ is calculated using GraphPad Software.

### 2.4 Cell cycle and apoptosis

K562 or HT29 cells were treated with increasing amounts of HDAC inhibitors in order to assess the biological response. In order to establish the effect of the HDAC inhibitors on the cell cycle and apoptosis the cells were fixed in 70% EtOH for 30 min, re-suspended in propidium iodide (PI: 50 µg/ml) with added RNAse (250 µg/ml) and incubated for 24 h at RT. The samples were analysed using a FACScan Cytometer (Beckton Dickinson). The tested compounds were able to determine a clear cell cycle modification and to induce apoptosis evaluated as sub-G1 analysis.

## Claims

1. Compounds of formula **(I)** 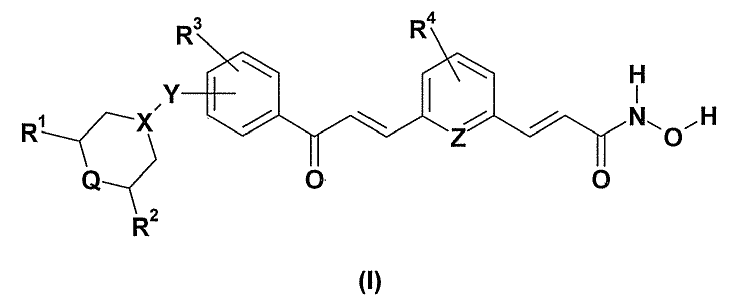 wherein:
**Q** is a bond, CH₂, **NR⁵** or oxygen;
**X** is CH or nitrogen;
**Y** is a bond, CH₂, oxygen or **NR⁶;**
**Z** is CH or nitrogen;
**R¹, R²** are, independently, hydrogen or C₁-C₆ alkyl;
**R³, R⁴** are, independently, hydrogen, halogen, C₁-C₆ alkyl, C₁-C₆ alkoxy, C₁- C₆ haloalkyl or C₁-C₆ haloalkoxy;
**R⁵** is hydrogen, C₁-C₆ alkyl, (CO)R⁷, phenyl or benzyl;
**R⁶** is hydrogen, C₁-C₆ alkyl or benzyl;
**R⁷** is hydrogen, C₁-C₆ alkyl, phenyl, benzyl, O**R⁸** or **NR⁹R¹⁰;**
**R⁸** is C₂-C₆ alkyl;
**R⁹** is hydrogen, C₁-C₆ alkyl, phenyl or benzyl;
**R¹⁰** is hydrogen, C₁-C₆ alkyl or benzyl;
and the pharmaceutically acceptable salts thereof.

2. Compounds according to claim 1, wherein:
**Q** is a bond, CH₂, **NR⁵** or oxygen;
**X** is CH or nitrogen;
**Y** is a bond or CH₂;
**Z** is CH or nitrogen;
**R¹, R²** are, independently, hydrogen or C₁-C₄ alkyl;
**R³, R⁴** are, independently, hydrogen, halogen, C₁-C₄ alkyl, C₁-C₄ alkoxy, C₁- C₄ haloalkyl or C₁-C₄ haloalkoxy;
**R⁵** is hydrogen, C₁-C₄ alkyl, (CO)**R⁷**, phenyl or benzyl;
**R⁷** is hydrogen, C₁-C₄ alkyl, phenyl, benzyl, O**R⁸** or **NR⁹R¹⁰;**
**R⁸** is C₂-C₄ alkyl;
**R⁹** is hydrogen, C₁-C₄ alkyl, phenyl or benzyl;
**R¹⁰** is hydrogen, C₁-C₄ alkyl or benzyl;
and the pharmaceutically acceptable salts thereof.

3. Compounds according to claim 1, wherein:
**Q** is a bond, CH₂, N**R⁵** or oxygen;
**X** is CH or nitrogen;
**Y** is a bond or CH₂;
**Z** is CH or nitrogen;
**R¹, R²** are, independently, hydrogen or C₁-C₂ alkyl;
**R³, R⁴** are, independently, hydrogen, fluoro, chloro, C₁-C₄ alkyl, C₁-C₄ alkoxy, CF₃ or C₁-C₂ haloalkoxy;
**R⁵** is hydrogen, C₁-C₂ alkyl, (CO)**R⁷**, phenyl or benzyl;
**R⁷** is hydrogen, C₁-C₂ alkyl, phenyl, benzyl, O**R⁸** or **NR⁹R¹⁰;**
**R⁸** is C₂-C₄ alkyl;
**R⁹** is hydrogen, C₁-C₂ alkyl, phenyl or benzyl;
**R¹⁰** is hydrogen, C₁-C₂ alkyl or benzyl;
and the pharmaceutically acceptable salts thereof.

4. Compounds according to claim 1, wherein:
**Q** is N**R⁵** or oxygen;
**X** is nitrogen;
**Y** is a bond or CH₂
**Z** is CH or nitrogen;
**R¹, R²** are, independently, hydrogen or C₁-C₂ alkyl;
**R³, R⁴** are, independently, hydrogen, halogen, C₁-C₄ alkyl, C₁-C₄ alkoxy, C₁- C₄ haloalkyl or C₁-C₄ haloalkoxy;
**R⁵** is hydrogen, C₁-C₆ alkyl or (CO)CH₃;
and the pharmaceutically acceptable salts thereof.

5. Compounds according to claim 1, selected from:
(E)-N-Hydroxy-3-(3-{(E)-3-[2-(4-methyl-piperazin-1-yl)-phenyl]-3-oxo-propenyl}-phenyl)-acrylamide;
(E)-N-Hydroxy-3-(3-{(E)-3-[2-(4-methyl-piperazin-1-ylmethyl)-phenyl]-3-oxo-propenyl}-phenyl)-acrylamide;
(E)-N-Hydroxy-3-(3-{(E)-3-[3-(4-methyl-piperazin-1-yl)-phenyl]-3-oxo-propenyl}-phenyl)-acrylamide;
(E)-3-(3-{(E)-3-[3-Chloro-5-(4-methyl-piperazin-1-yl)-phenyl]-3-oxo-propenyl}-phenyl)-N-hydroxy-acrylamide;
(E)-N-Hydroxy-3-(3-{(E)-3-[3-(4-methyl-piperazin-1-ylmethyl)-phenyl]-3-oxo-propenyl}-phenyl)-acrylamide;
(E)-N-Hydroxy-3-(3-{(E)-3-[4-(4-methyl-piperazin-1-yl)-phenyl]-3-oxo-propenyl}-phenyl)-acrylamide;
(E)-N-Hydroxy-3-(3-{(E)-3-[4-(4-methyl-piperazin-1-ylmethyl)-phenyl]-3-oxo-propenyl}-phenyl)-acrylamide;
(E)-N-Hydroxy-3-(6-{(E)-3-[2-(4-methyl-piperazin-1-yl)-phenyl]-3-oxo-propenyl}-pyridin-2-yl)-acrylamide;
(E)-N-Hydroxy-3-(6-{(E)-3-[2-(4-methyl-piperazin-1-ylmethyl)-phenyl]-3-oxo-propenyl}-pyridin-2-yl)-acrylamide;
(E)-N-Hydroxy-3-(6-{(E)-3-[3-(4-methyl-piperazin-1-yl)-phenyl]-3-oxo-propenyl}-pyridin-2-yl)-acrylamide;
(E)-3-(6-{(E)-3-[3-Chloro-5-(4-methyl-piperazin-1-yl)-phenyl]-3-oxo-propenyl}-pyridin-2-yl)-N-hydroxy-acrylamide;
(E)-N-Hydroxy-3-(6-{(E)-3-[3-(4-methyl-piperazin-1-ylmethyl)-phenyl]-3-oxo-propenyl}-pyridin-2-yl)-acrylamide;
(E)-N-Hydroxy-3-(6-{(E)-3-[4-(4-methyl-piperazin-1-yl)-phenyl]-3-oxo-propenyl}-pyridin-2-yl)-acrylamide;
(E)-N-Hydroxy-3-(6-{(E)-3-[4-(4-methyl-piperazin-1-ylmethyl)-phenyl]-3-oxo-propenyl}-pyridin-2-yl)-acrylamide;
(E)-3-(3-{(E)-3-[2-(4-Acetyl-piperazin-1-yl)-phenyl]-3-oxo-propenyl}-phenyl)-N-hydroxy-acrylamide;
(E)-3-(3-{(E)-3-[3-(4-Acetyl-piperazin-1-yl)-phenyl]-3-oxo-propenyl}-phenyl)-N-hydroxy-acrylamide;
(E)-3-(3-{(E)-3-[4-(4-Acetyl-piperazin-1-yl)-phenyl]-3-oxo-propenyl}-phenyl)-N-hydroxy-acrylamide;
(E)-3-(6-{(E)-3-[2-(4-Acetyl-piperazin-1-yl)-phenyl]-3-oxo-propenyl}-pyridin-2-yl)-N-hydroxy-acrylamide;
(E)-3-(6-{(E)-3-[3-(4-Acetyl-piperazin-1-yl)-phenyl]-3-oxo-propenyl}-pyridin-2-yl)-N-hydroxy-acrylamide;
(E)-3-(6-{(E)-3-[4-(4-Acetyl-piperazin-1-yl)-phenyl]-3-oxo-propenyl}-pyridin-2-yl)-N-hydroxy-acrylamide;
(E)-3-(3-{(E)-3-[2-(4-Acetyl-piperazin-1-ylmethyl)-phenyl]-3-oxo-propenyl}-phenyl)-N-hydroxy-acrylamide;
(E)-3-(3-{(E)-3-[3-(4-Acetyl-piperazin-1-ylmethyl)-phenyl]-3-oxo-propenyl}-phenyl)-N-hydroxy-acrylamide;
(E)-3-(3-{(E)-3-[4-(4-Acetyl-piperazin-1-ylmethyl)-phenyl]-3-oxo-propenyl}-phenyl)-N-hydroxy-acrylamide;
(E)-3-(6-{(E)-3-[2-(4-Acetyl-piperazin-1-ylmethyl)-phenyl]-3-oxo-propenyl}-pyridin-2-yl)-N-hydroxy-acrylamide;
(E)-3-(6-{(E)-3-[3-(4-Acetyl-piperazin-1-ylmethyl)-phenyl]-3-oxo-propenyl}-pyridin-2-yl)-N-hydroxy-acrylamide;
(E)-3-(6-{(E)-3-[4-(4-Acetyl-piperazin-1-ylmethyl)-phenyl]-3-oxo-propenyl}-pyridin-2-yl)-N-hydroxy-acrylamide;
(E)-3-(3-{(E)-3-[2-(4-Phenyl-piperazin-1-ylmethyl)-phenyl]-3-oxo-propenyl}-phenyl)-N-hydroxy-acrylamide;
(E)-3-(3-{(E)-3-[3-(4-Phenyl-piperazin-1-ylmethyl)-phenyl]-3-oxo-propenyl}-phenyl)-N-hydroxy-acrylamide;
(E)-3-(3-{(E)-3-[4-(4-Phenyl-piperazin-1-ylmethyl)-phenyl]-3-oxo-propenyl}-phenyl)-N-hydroxy-acrylamide;
(E)-3-(6-{(E)-3-[2-(4-Phenyl-piperazin-1-ylmethyl)-phenyl]-3-oxo-propenyl}-pyridin-2-yl)-N-hydroxy-acrylamide;
(E)-3-(6-{(E)-3-[3-(4-Phenyl-piperazin-1-ylmethyl)-phenyl]-3-oxo-propenyl}-pyridin-2-yl)-N-hydroxy-acrylamide;
(E)-3-(6-{(E)-3-[4-(4-Phenyl-piperazin-1-ylmethyl)-phenyl]-3-oxo-propenyl}-pyridin-2-yl)-N-hydroxy-acrylamide;
(E)-3-(3-{(E)-3-[2-(4-Benzyl-piperazin-1-yl)-phenyl]-3-oxo-propenyl}-phenyl)-N-hydroxy-acrylamide;
(E)-3-(3-{(E)-3-[3-(4-Benzyl-piperazin-1-yl)-phenyl]-3-oxo-propenyl}-phenyl)-N-hydroxy-acrylamide;
(E)-3-(3-{(E)-3-[4-(4-Benzyl-piperazin-1-yl)-phenyl]-3-oxo-propenyl}-phenyl)-N-hydroxy-acrylamide;
(E)-3-(6-{(E)-3-[2-(4-Benzyl-piperazin-1-yl)-phenyl]-3-oxo-propenyl}-pyridin-2-yl)-N-hydroxy-acrylamide;
(E)-3-(6-{(E)-3-[3-(4-Benzyl-piperazin-1-yl)-phenyl]-3-oxo-propenyl}-pyridin-2-yl)-N-hydroxy-acrylamide;
(E)-3-(6-{(E)-3-[4-(4-Benzyl-piperazin-1-yl)-phenyl]-3-oxo-propenyl}-pyridin-2-yl)-N-hydroxy-acrylamide;
(E)-3-(3-{(E)-3-[2-(4-Benzyl-piperazin-1-ylmethyl)-phenyl]-3-oxo-propenyl}-phenyl)-N-hydroxy-acrylamide;
(E)-3-(3-{(E)-3-[3-(4-Benzyl-piperazin-1-ylmethyl)-phenyl]-3-oxo-propenyl}-phenyl)-N-hydroxy-acrylamide;
(E)-3-(3-{(E)-3-[4-(4-Benzyl-piperazin-1-ylmethyl)-phenyl]-3-oxo-propenyl}-phenyl)-N-hydroxy-acrylamide;
(E)-3-(6-{(E)-3-[2-(4-Benzyl-piperazin-1-ylmethyl)-phenyl]-3-oxo-propenyl}-pyridin-2-yl)-N-hydroxy-acrylamide;
(E)-3-(6-{(E)-3-[3-(4-Benzyl-piperazin-1-ylmethyl)-phenyl]-3-oxo-propenyl}-pyridin-2-yl)-N-hydroxy-acrylamide;
(E)-3-(6-{(E)-3-[4-(4-Benzyl-piperazin-1-ylmethyl)-phenyl]-3-oxo-propenyl}-pyridin-2-yl)-N-hydroxy-acrylamide;
(E)-N-Hydroxy-3-(3-{(E)-3-oxo-3-[2-((3R,5S)-3,4,5-trimethyl-piperazin-1-yl)-phenyl]-propenyl}-phenyl)-acrylamide;
(E)-N-Hydroxy-3-(3-{(E)-3-oxo-3-[3-((3R,5S)-3,4,5-trimethyl-piperazin-1-yl)-phenyl]-propenyl}-phenyl)-acrylamide;
(E)-N-Hydroxy-3-(3-{(E)-3-oxo-3-[4-((3R,5S)-3,4,5-trimethyl-piperazin-1-yl)-phenyl]-propenyl}-phenyl)-acrylamide;
(E)-N-Hydroxy-3-(6-{(E)-3-oxo-3-[2-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-yl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-yl)-phenyl]-propenyl}-pyridin-2-yl)-acrylamide;
(E)-N-Hydroxy-3-(3-{(E)-3-oxo-3-[2-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[3-((3R,5S)-3,4,5-trimethyl-piperazin-1-ylmethyl)-phenyl]-propenyl}-phenyl)-acrylamide;
(E)-N-Hydroxy-3-(3-{(E)-3-oxo-3-[4-((3R,5S)-3,4,5-trimethyl-piperazin-1-ylmethyl)-phenyl]-propenyl}-phenyl)-acrylamide;
(E)-N-Hydroxy-3-(6-{(E)-3-oxo-3-[2-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[3-((3R,5S)-3,4,5-trimethyl-piperazin-1-ylmethyl)-phenyl]-propenyl}-pyridin-2-yl)-acrylamide;
(E)-N-Hydroxy-3-(6-{(E)-3-oxo-3-[4-((3R,5S)-3,4,5-trimethyl-piperazin-1-ylmethyl)-phenyl]-propenyl}-pyridin-2-yl)-acrylamide;
(E)-3-(3-{(E)-3-[2-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-yl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-yl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-yl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-yl)-phenyl]-3-oxo-propenyl}-pyridin-2-yl)-N-hydroxy-acrylamide;
(E)-3-(3-{(E)-3-[2-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[3-((3R,5S)-4-Acetyl-3,5-dimethyl-piperazin-1-ylmethyl)-phenyl]-3-oxo-propenyl}-phenyl)-N-hydroxy-acrylamide;
(E)-3-(3-{(E)-3-[4-((3R,5S)-4-Acetyl-3,5-dimethyl-piperazin-1-ylmethyl)-phenyl]-3-oxo-propenyl}-phenyl)-N-hydroxy-acrylamide;
(E)-3-(6-{(E)-3-[2-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-4-Acetyl-3,5-dimethyl-piperazin-1-ylmethyl)-phenyl]-3-oxo-propenyl}-pyridin-2-yl)-N-hydroxy-acrylamide;
(E)-N-Hydroxy-3-{3-[(E)-3-(2-morpholin-4-yl-phenyl)-3-oxo-propenyl]-phenyl}-acrylamide;
(E)-N-Hydroxy-3-{3-[(E)-3-(3-morpholin-4-yl-phenyl)-3-oxo-propenyl]-phenyl}-acrylamide;
(E)-N-Hydroxy-3-{3-[(E)-3-(4-morpholin-4-yl-phenyl)-3-oxo-propenyl]-phenyl}-acrylamide;
(E)-N-Hydroxy-3-{6-[(E)-3-(2-morpholin-4-yl-phenyl)-3-oxo-propenyl]-pyridin-2-yl}-acrylamide;
(E)-N-Hydroxy-3-{6-[(E)-3-(3-morpholin-4-yl-phenyl)-3-oxo-propenyl]-pyridin-2-yl}-acrylamide;
(E)-N-Hydroxy-3-{6-[(E)-3-(4-morpholin-4-yl-phenyl)-3-oxo-propenyl]-pyridin-2-yl}-acrylamide;
(E)-N-Hydroxy-3-{3-[(E)-3-(2-morpholin-4-ylmethyl-phenyl)-3-oxo-propenyl]-phenyl}-acrylamide;
(E)-N-Hydroxy-3-{3-[(E)-3-(3-morpholin-4-ylmethyl-phenyl)-3-oxo-propenyl]-phenyl}-acrylamide;
(E)-N-Hydroxy-3-{3-[(E)-3-(4-morpholin-4-ylmethyl-phenyl)-3-oxo-propenyl]-phenyl}-acrylamide;
(E)-N-Hydroxy-3-{6-[(E)-3-(2-morpholin-4-ylmethyl-phenyl)-3-oxo-propenyl]-pyridin-2-yl}-acrylamide;
(E)-N-Hydroxy-3-{6-[(E)-3-(3-morpholin-4-ylmethyl-phenyl)-3-oxo-propenyl]-pyridin-2-yl}-acrylamide;
(E)-N-Hydroxy-3-{6-[(E)-3-(4-morpholin-4-ylmethyl-phenyl)-3-oxo-propenyl]-pyridin-2-yl}-acrylamide;
(E)-N-Hydroxy-3-{3-[(E)-3-oxo-3-(2-piperazin-1-yl-phenyl)-propenyl]-phenyl}-acrylamide;
(E)-N-Hydroxy-3-{3-[(E)-3-oxo-3-(3-piperazin-1-yl-phenyl)-propenyl]-phenyl}-acrylamide;
(E)-N-Hydroxy-3-{3-[(E)-3-oxo-3-(4-piperazin-1-yl-phenyl)-propenyl]-phenyl}-acrylamide;
(E)-N-Hydroxy-3-{6-[(E)-3-oxo-3-(2-piperazin-1-yl-phenyl)-propenyl]-pyridin-2-yl}-acrylamide;
(E)-N-Hydroxy-3-{6-[(E)-3-oxo-3-(3-piperazin-1-yl-phenyl)-propenyl]-pyridin-2-yl}-acrylamide;
(E)-N-Hydroxy-3-{6-[(E)-3-oxo-3-(4-piperazin-1-yl-phenyl)-propenyl]-pyridin-2-yl}-acrylamide;
(E)-N-Hydroxy-3-{3-[(E)-3-oxo-3-(2-piperazin-1-ylmethyl-phenyl)-propenyl]-phenyl}-acrylamide;
(E)-N-Hydroxy-3-{3-[(E)-3-oxo-3-(3-piperazin-1-ylmethyl-phenyl)-propenyl]-phenyl}-acrylamide;
(E)-N-Hydroxy-3-{3-[(E)-3-oxo-3-(4-piperazin-1-ylmethyl-phenyl)-propenyl]-phenyl}-acrylamide;
(E)-N-Hydroxy-3-{6-[(E)-3-oxo-3-(2-piperazin-1-ylmethyl-phenyl)-propenyl]-pyridin-2-yl}-acrylamide;
(E)-N-Hydroxy-3-{6-[(E)-3-oxo-3-(3-piperazin-1-ylmethyl-phenyl)-propenyl]-pyridin-2-yl}-acrylamide;
(E)-N-Hydroxy-3-{6-[(E)-3-oxo-3-(4-piperazin-1-ylmethyl-phenyl)-propenyl]-pyridin-2-yl}-acrylamide;
(E)-3-(3-{(E)-3-[2-((3R,5S)-3,5-Dimethyl-piperazin-1-yl)-phenyl]-3-oxo-propenyl}-phenyl)-N-hydroxy-acrylamide;
(E)-3-(3-{(E)-3-[3-((3R,5S)-3,5-Dimethyl-piperazin-1-yl)-phenyl]-3-oxo-propenyl}-phenyl)-N-hydroxy-acrylamide;
(E)-3-(3-{(E)-3-[4-((3R,5S)-3,5-Dimethyl-piperazin-1-yl)-phenyl]-3-oxo-propenyl}-phenyl)-N-hydroxy-acrylamide;
(E)-3-(6-{(E)-3-[2-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-yl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-yl)-phenyl]-3-oxo-propenyl}-pyridin-2-yl)-N-hydroxy-acrylamide;
(E)-3-(3-{(E)-3-[2-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[3-((3R,5S)-3,5-Dimethyl-piperazin-1-ylmethyl)-phenyl]-3-oxo-propenyl}-phenyl)-N-hydroxy-acrylamide;
(E)-3-(3-{(E)-3-[4-((3R,5S)-3,5-Dimethyl-piperazin-1-ylmethyl)-phenyl]-3-oxo-propenyl}-phenyl)-N-hydroxy-acrylamide;
(E)-3-(6-{(E)-3-[2-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[3-((3R,5S)-3,5-Dimethyl-piperazin-1-ylmethyl)-phenyl]-3-oxo-propenyl}-pyridin-2-yl)-N-hydroxy-acrylamide;
(E)-3-(6-{(E)-3-[4-((3R,5S)-3,5-Dimethyl-piperazin-1-ylmethyl)-phenyl]-3-oxo-propenyl}-pyridin-2-yl)-N-hydroxy-acrylamide.

6. Compounds of formula **(I)** 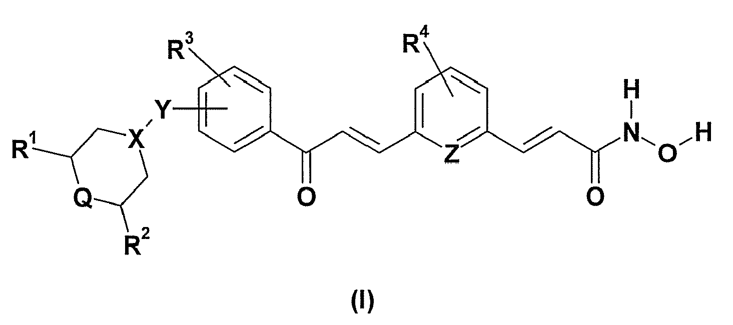 wherein:
**Q** is a bond, CH₂, **NR⁵** or oxygen;
**X** is CH or nitrogen;
**Y** is a bond, CH₂, oxygen or **NR⁶;**
**Z** is CH or nitrogen;
**R¹, R²** are, independently, hydrogen or C₁-C₆ alkyl;
**R³, R⁴** are, independently, hydrogen, halogen, C₁-C₆ alkyl, C₁-C₆ alkoxy, C₁- C₆ haloalkyl or C₁-C₆ haloalkoxy;
**R⁵** is hydrogen, C₁-C₆ alkyl, (CO)**R⁷**, phenyl or benzyl;
**R⁶** is hydrogen, C₁-C₆ alkyl or benzyl;
**R⁷** is hydrogen, C₁-C₆ alkyl, phenyl, benzyl, O**R⁸** or **NR⁹R¹⁰;**
**R⁸** is C₂-C₆ alkyl;
**R⁹** is hydrogen, C₁-C₆ alkyl, phenyl or benzyl;
**R¹⁰** is hydrogen, C₁-C₆ alkyl or benzyl;
and the pharmaceutically acceptable salts thereof,
for use in therapy.

7. Compounds according to claim 6, for use in the treatment or prevention of diseases linked to the disregulation of histone deacetylase activity.

8. Pharmaceutical composition containing one or more compounds of formula **(I)** 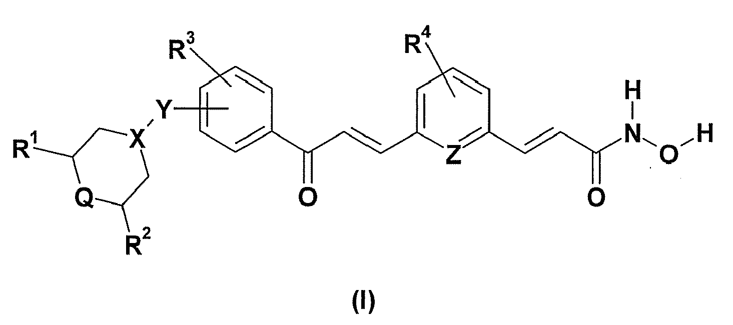 wherein:
**Q** is a bond, CH₂, **NR⁵** or oxygen;
X is CH or nitrogen;
**Y** is a bond, CH₂, oxygen or **NR⁶;**
Z is CH or nitrogen;
**R¹, R²** are, independently, hydrogen or C₁-C₆ alkyl;
**R³, R⁴** are, independently, hydrogen, halogen, C₁-C₆ alkyl, C₁-C₆ alkoxy, C₁- C₆ haloalkyl or C₁-C₆ haloalkoxy;
**R⁵** is hydrogen, C₁-C₆ alkyl, (CO)**R⁷**, phenyl or benzyl;
**R⁶** is hydrogen, C₁-C₆ alkyl or benzyl;
**R⁷** is hydrogen, C₁-C₆ alkyl, phenyl, benzyl, O**R⁸** or **NR⁹R¹⁰;**
**R⁸** is C₂-C₆ alkyl;
**R⁹** is hydrogen, C₁-C₆ alkyl, phenyl or benzyl;
**R¹⁰** is hydrogen, C₁-C₆ alkyl or benzyl;
and the pharmaceutically acceptable salts thereof,
in association with pharmaceutically acceptable excipients and diluents.

9. Pharmaceutical composition according to claim 8, wherein the compound of formula **(I)** is present in association with a further active principle.

10. Pharmaceutical composition according to claim 9, wherein the further active principle is an antitumour agent.

11. Use of one or more compounds of formula **(I)** 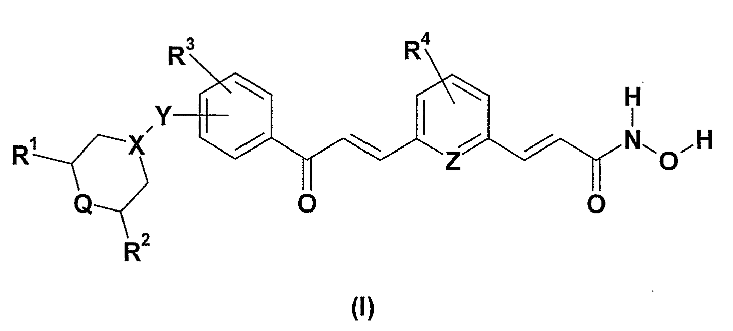 wherein:
**Q** is a bond, CH₂, **NR⁵** or oxygen;
**X** is CH or nitrogen;
**Y** is a bond, CH₂, oxygen or **NR⁶;**
**Z** is CH or nitrogen;
**R¹, R²** are, independently, hydrogen or C₁-C₆ alkyl;
**R³, R⁴** are, independently, hydrogen, halogen, C₁-C₆ alkyl, C₁-C₆ alkoxy, C₁- C₆ haloalkyl or C₁-C₆ haloalkoxy;
**R⁵** is hydrogen, C₁-C₆ alkyl, (CO)**R⁷**, phenyl or benzyl;
**R⁶** is hydrogen, C₁-C₆ alkyl or benzyl;
**R⁷** is hydrogen, C₁-C₆ alkyl, phenyl, benzyl, O**R⁸** or **NR⁹R¹⁰;**
**R⁸** is C₂-C₆ alkyl;
**R⁹** is hydrogen, C₁-C₆ alkyl, phenyl or benzyl;
**R¹⁰** is hydrogen, C₁-C₆ alkyl or benzyl;
and the pharmaceutically acceptable salts thereof,
for the manufacture of a medicament for treating or preventing diseases linked to the disregulation of histone deacetylase activity.

12. Process for preparing the compounds of formula **(I)** as defined in claims 1-5, comprising:
(i) reacting a compound of formula **(1)** with a compound of formula **(2)** 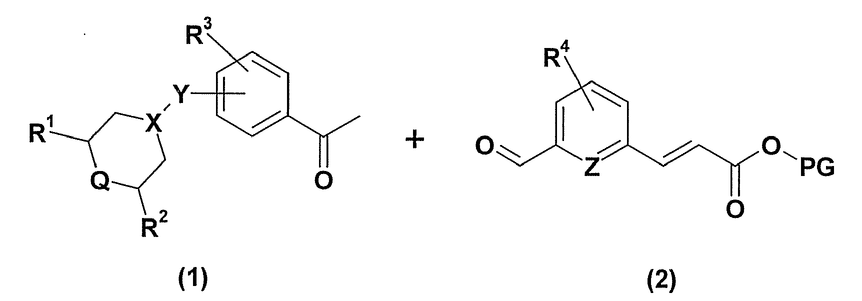 wherein **Q, X, Y, Z, R¹, R², R³, R⁴** are as defined in claim 1 and PG is a protecting group, obtaining the corresponding compound of formula **(3)** 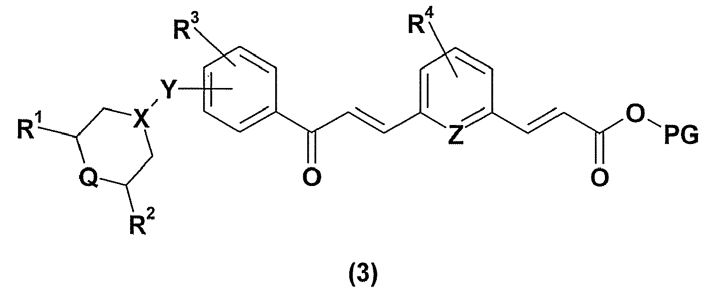
(ii) removing the PG group from the compound of formula **(3),**
(iii) treating the product of step (ii) with a protected hydroxylamine
(iv) removing of the hydroxylamine-protecting group from the product of step (iii), thereby obtaining the compound of formula **(I).**
